# EUROPEAN PATENT APPLICATION

(11) **EP 3 257 941 A1**
(43) Date of publication of application: **20.12.2017**
(21) Application number: 16749280.0
(22) Date of filing: 10.02.2016
(51) Int. Cl.: C12N 15/09, C07K 14/00, C07K 19/00, C12N 1/21, C12N 9/00, C12P 5/00, C12P 5/02, C12R 1/145

(54) **NUCLEIC ACID, FUSION PROTEIN, RECOMBINED CELL, AND ISOPRENE OR CYCLIC TERPENE PRODUCTION METHOD**

(30) Priority: 13.02.2015 JP 2015026156; 06.11.2015 JP 2015218114
(71) Applicant: Sekisui Chemical Co., Ltd., Osaka-shi, Osaka 530-8565 (JP)
(72) Inventor: FURUTANI, Masahiro, Tsukuba-shi Ibaraki 300-4292 (JP); KAWABATA, Kazufumi, Tsukuba-shi Ibaraki 300-4292 (JP); NISHIYAMA, Norihide, Tsukuba-shi Ibaraki 300-4292 (JP)
(74) Representative: Ter Meer Steinmeister & Partner
(86) International application number: PCT/JP2016/053977
(87) International publication number: WO 2016/129637

(57) **Abstract**

Provided is a nucleic acid encoding a fusion protein, the fusion protein including a first protein selected from the group consisting of isoprene synthase and cyclic terpene synthase, and a FKBP family protein linked to the first protein. Provided is a fusion protein encoded by the nucleic acid. Provided is a recombinant cell including the nucleic acid and expressing the fusion protein. Further provided is a recombinant cell including a first nucleic acid encoding the first protein and a second nucleic acid encoding the FKBP family protein, and expressing the first protein and the FKBP family protein. As a host cell, a syngas-assimilating bacterium or a methanol assimilating bacterium can be used.

## Description

### TECHNICAL FIELD

The present invention relates to a nucleic acid, a fusion protein, a recombinant cell, and a method for producing isoprene or cyclic terpene. In particular, the present invention relates to a nucleic acid encoding a fusion protein that includes isoprene synthase or cyclic terpene synthase and a FKBP family protein, the fusion protein, a recombinant cell including the nucleic acid and the like, and a method for producing isoprene or cyclic terpene using the recombinant cell.

### BACKGROUND ART

Isoprene is a monomer raw material for synthetic polyisoprene, and is an important material, in particular, in the tire industry. In recent years, development and commercialization has been steadily progressing in the technique for conversion from a production process of basic chemicals depending on petroleum to a production process from renewable resources such as plant resources.

Isoprene synthase (EC 4.2.3.27) has action of converting dimethylallyl diphosphate (DMAPP) as an isomer of isopentenyl diphosphate (IPP) into isoprene.

A method for producing isoprene using a recombinant cell (a recombinant) is known. For example, in the inventions described in Patent Documents 1 and 2, isoprene is produced using a recombinant cell into which a nucleic acid (gene) encoding isoprene synthase has been introduced. In detail, the recombinant cell is cultured using methanol or syngas (synthesis gas) as a carbon source, and isoprene is obtained from the cultured product. Furthermore, a production technique by recombinant *Escherichia coli* using sugar as a raw material is known (see, for example, Patent Documents 3 and 4).

Terpene is a generic name of compounds having 10 or more carbon atoms in which two or more molecules of isopentenyl diphosphate (IPP) as an isoprene unit (C5) are linked to each other by the action of prenyltransferase. Terpenes are classified into monoterpene (C10), sesquiterpene (C15), diterpene (C20), triterpene (C30), tetraterpene (C40), and the like, depending on the number of isoprene units. Inter alia, many cyclic monoterpenes, cyclic sesquiterpenes, and cyclic diterpenes are useful as perfume raw materials, cosmetic raw materials, pharmaceutical intermediates, adhesive raw materials, and high function resin raw materials.

An enzyme for producing cyclic terpene using linear terpene as a substrate is referred to as cyclic terpene synthase. Cyclic terpene is synthesized by cyclization of linear terpene by the action of the cyclic terpene synthase. For example, cyclic monoterpene is synthesized as follows, that is, for example, geranyl diphosphate (GPP) generates by biomolecular binding of IPP, and is formed into a cyclic structure by action of cyclic monoterpene synthase. The cyclic sesquiterpene is synthesized by cyclization of farnesyl diphosphate (C15: FPP) as a precursor by the action of cyclic sesquiterpene synthase. The cyclic diterpene is synthesized by cyclization of geranylgeranyl diphosphate (C20: GGPP) as a precursor by the action of cyclic diterpene synthase.

Well-known examples of the method for producing cyclic terpene using a recombinant cell (a recombinant) include a method for producing β-phellandrene as one type of cyclic monoterpene (Patent Document 5). In this method, β-phellandrene is produced by converting geranyl diphosphate (GPP) or neryl diphosphate (NPP) into β-phellandrene by using a recombinant cell into which a nucleic acid encoding β-phellandrene synthase has been introduced. The β-phellandrene has a β-phellandrene conjugated diene structure, and therefore is useful natural monomer for polymerization.

### PRIOR ART DOCUMENTS

### PATENT DOCUMENTS

Patent Document 1: WO2014/065271
Patent Document 2: WO2014/104202
Patent Document 3: WO2009/076676
Patent Document 4: WO2009/132220
Patent Document 5: JP 2014-76042 A

### DISCLOSURE OF INVENTION

### TECHNICAL PROBLEM

Most of the currently available isoprene synthase and cyclic terpene synthase are derived from plants. Therefore, when they are expressed, in particular, in a bacterial host cell, three-dimensional structure cannot be formed successfully in the cytoplasm, causing problems such as degradation by host protease, or insolubilization (Patent Documents 3 and 4). In order to improve productivity of isoprene or cyclic terpene, improvement of stability of isoprene synthase and cyclic terpene synthase in a recombinant cell has been demanded.

An object of the present invention is to provide a technique for improving the stability of isoprene synthase and cyclic terpene synthase in a recombinant cell, and improving the productivity of isoprene and cyclic terpene by a recombinant cell.

### SOLUTION TO PROBLEM

The inventors have succeeded in improving the stability of isoprene synthase and cyclic terpene synthase in a recombinant cell by using a FKBP family protein as one type of molecular chaperone. Then, the inventors have succeeded in improving the productivity of isoprene and cyclic terpene by using the recombinant cell, reaching the completion of the present invention.

One aspect of the present invention relates to a nucleic acid encoding a fusion protein, the fusion protein including: a first protein selected from the group consisting of isoprene synthase and cyclic terpene synthase; and a FKBP family protein linked to the first protein.

The nucleic acid of this aspect encodes a fusion protein (chimeric protein) which includes a first protein selected from the group consisting of isoprene synthase and cyclic terpene synthase, and a FKBP family protein linked to the first protein. Since in the fusion protein, isoprene synthase or cyclic terpene synthase is stabilized by the action of FKBP family protein adjacent thereto in the same molecule, the isoprene synthase activity or the cyclic terpene synthase activity is exhibited more stably. Therefore, for example, when the fusion protein is expressed in a recombinant cell into which the nucleic acid of this aspect has been introduced, degradation by host protease, or insolubilization is less likely to occur in the recombinant cell as compared with the case where isoprene synthase or cyclic terpene synthase is singly used.

Preferably, the first protein is isoprene synthase.

The nucleic acid of this aspect encodes a fusion protein which includes isoprene synthase and a FKBP family protein linked thereto. Since in the fusion protein, isoprene synthase is stabilized by the action of FKBP family protein adjacent thereto in the same molecule, the isoprene synthase activity is exhibited more stably. Therefore, for example, when the fusion protein is expressed in a recombinant cell into which the nucleic acid of this aspect has been introduced, degradation by host protease, or insolubilization is less likely to occur in the recombinant cell as compared with the case where isoprene synthase is singly used.

Preferably, the isoprene synthase is any one of the following (a-1) to (a-3):
(a-1) a protein consisting of the amino acid sequence of SEQ ID NO: 2,
(a-2) a protein consisting of the amino acid sequence in which 1 to 20 amino acids are deleted, substituted or added in the amino acid sequence of SEQ ID NO: 2, and having isoprene synthase activity, and
(a-3) a protein consisting of an amino acid sequence having homology of 90% or more with the amino acid sequence of SEQ ID NO: 2, and having isoprene synthase activity.

Preferably, the first protein is cyclic monoterpene synthase.

Preferably, the cyclic monoterpene synthase is phellandrene synthase.

Preferably, the cyclic monoterpene synthase is any one of the following (b-1) to (b-3):
(b-1) a protein consisting of the amino acid sequence of SEQ ID NO: 4,
(b-2) a protein consisting of the amino acid sequence in which 1 to 20 amino acids are deleted, substituted or added in the amino acid sequence of SEQ ID NO: 4, and having β-phellandrene synthase activity, and
(b-3) a protein consisting of an amino acid sequence having homology of 90% or more with the amino acid sequence of SEQ ID NO: 4, and having β-phellandrene synthase activity.

Preferably, the first protein is cyclic sesquiterpene synthase.

Preferably, the cyclic sesquiterpene synthase is any one of the following (c-1) to (c-3):
(c-1) a protein consisting of the amino acid sequence of SEQ ID NO: 6,
(c-2) a protein consisting of the amino acid sequence in which 1 to 20 amino acids are deleted, substituted or added in the amino acid sequence of SEQ ID NO: 6, and having trichodiene synthase activity, and
(c-3) a protein consisting of an amino acid sequence having homology of 90% or more with the amino acid sequence of SEQ ID NO: 6, and having trichodiene synthase activity.

Preferably, the first protein is cyclic diterpene synthase.

Preferably, the first protein is derived from prokaryote.

Preferably, the FKBP family protein is derived from prokaryote.

Preferably, the FKBP family protein is derived from archaea.

Preferably, the FKBP family protein has a molecular weight of 20,000 or less.

Preferably, the FKBP family protein is any one of the following (d-1) to (d-3):
(d-1) a protein consisting of the amino acid sequence of SEQ ID NO: 8,
(d-2) a protein consisting of the amino acid sequence in which 1 to 20 amino acids are deleted, substituted or added in the amino acid sequence of SEQ ID NO: 8, and having activity as the FKBP family protein, and
(d-3) a protein consisting of the amino acid sequence having homology of 90% or more with the amino acid sequence of SEQ ID NO: 8, and having activity as the FKBP family protein.

Preferably, the FKBP family protein belongs to a trigger factor.

Preferably, the FKBP family protein is linked to an N-terminal side of the first protein.

Preferably, the fusion protein further includes a secretion signal sequence.

One aspect of the present invention relates to a fusion protein encoded by the nucleic acid mentioned above.

One aspect of the present invention relates to a recombinant cell being a bacterium, including the above-mentioned nucleic acid, and expressing the fusion protein.

The recombinant cell of this aspect relates to a recombinant cell that is a bacterium, and expresses the fusion protein. According to the recombinant cell of this aspect, since the isoprene synthase activity or the cyclic terpene synthase activity is exhibited by the fusion protein, more stabilized isoprene synthase activity or cyclic terpene synthase activity is obtained.

One aspect of the present invention relates to a recombinant cell being a bacterium, including: a first nucleic acid encoding a first protein selected from the group consisting of isoprene synthase and cyclic terpene synthase; and a second nucleic acid encoding a FKBP family protein, wherein the recombinant cell expresses the isoprene synthase or the cyclic terpene synthase, and expresses the FKBP family protein.

The recombinant cell of this aspect is a bacterial recombinant cell that includes a first nucleic acid encoding isoprene synthase or cyclic terpene synthase, and a second nucleic acid encoding a FKBP family protein. According to the recombinant cell of this aspect, since the isoprene synthase or the cyclic terpene synthase is stabilized by a coexistent FKBP family protein, more stabilized isoprene synthase activity or cyclic terpene synthase activity is obtained.

Preferably, the first protein is isoprene synthase, and and the recombinant cell expresses the isoprene synthase and the FKBP family protein.

The recombinant cell of this aspect includes a first nucleic acid encoding isoprene synthase, and a second nucleic acid encoding a FKBP family protein. According to the recombinant cell of this aspect, since the isoprene synthase is stabilized by a coexistent FKBP family protein, more stabilized isoprene synthase activity is obtained.

Preferably, the first protein is cyclic monoterpene synthase.

Preferably, the cyclic monoterpene synthase is phellandrene synthase.

Preferably, the first protein is cyclic sesquiterpene synthase.

Preferably, the first protein is cyclic diterpene synthase.

Preferably, the recombinant cell has a capability of assimilating a C1 compound.

Preferably, the recombinant cell has a capability of assimilating methanol or methane.

Preferably, the recombinant cell has a capability of assimilating carbon monoxide or carbon dioxide.

Preferably, the recombinant cell is an anaerobic bacterium.

Preferably, the recombinant cell is a *Clostridium* bacterium or a *Moorella* bacterium.

One aspect of the present invention relates to a method for producing isoprene or cyclic terpene, the method including: bringing gas that includes carbon dioxide and hydrogen into contact with the recombinant cell mentioned above; and allowing the recombinant cell to produce isoprene or cyclic terpene from carbon dioxide.

This aspect relates to a method for producing isoprene or cyclic terpene. The method includes bringing gas that includes carbon dioxide and hydrogen into contact with the recombinant cell above, and allowing the recombinant cell to produce isoprene or cyclic terpene from carbon dioxide. According to this aspect, since the isoprene synthase or cyclic terpene synthase expressed in the recombinant cell is stabilized by the FKBP family protein, isoprene or cyclic terpene is produced with high efficiency.

Preferably, the gas includes carbon monoxide, carbon dioxide and hydrogen, and the method allows the recombinant cell to produce isoprene or cyclic terpene from carbon monoxide and carbon dioxide.

This aspect also relates to a method for producing isoprene or cyclic terpene. The method includes bringing gas that includes carbon monoxide, carbon dioxide and hydrogen into contact with the above-mentioned recombinant cell, and allowing the recombinant cell to produce isoprene or cyclic terpene from carbon monoxide and carbon dioxide. Also, according to this aspect, since the isoprene synthase or cyclic terpene synthase expressed in the recombinant cell is stabilized by the FKBP family protein, isoprene or cyclic terpene is produced with high efficiency.

Preferably, isoprene or cyclic terpene released to the outside of the recombinant cell is collected.

Preferably, the isoprene or cyclic terpene is collected by a solid phase adsorption process.

Preferably, the isoprene or cyclic terpene is collected by a solvent absorption process.

### EFFECT OF INVENTION

The present invention remarkably improves the productivity in production of isoprene or cyclic terpene by a recombinant cell.

### BRIEF DESCRIPTION OF DRAWINGS

Fig. 1 is an explanatory diagram showing an acetyl CoA pathway and a methanol pathway.
Fig. 2 is an explanatory diagram showing a carbon anabolic metabolic pathway via formaldehyde.
Fig. 3 is an explanatory diagram showing a structure of a pSKCL vector.

### BEST MODE FOR CARRYING OUT THE INVENTION

A nucleic acid (gene) of the present invention encodes a fusion protein (chimeric protein) in which isoprene synthase or cyclic terpene synthase is linked to a FKBP family protein. In other words, the nucleic acid of the present invention is a fusion gene (chimeric gene) in which a gene encoding isoprene synthase or cyclic terpene synthase and a gene encoding the FKBP family protein are linked to each other.

The FKBP family protein is a FK506 binding protein (FKBP), and has Peptidyl-prolyl cis-trans isomerase (hereinafter, referred to as "PPIase") activity and molecular chaperone activity. The structures or classifications of the FKBP family proteins (FKBP-type PPIase) are described in, for example, WO2004/001041 and WO2005/063964.

Hereinafter, the FKBP family protein is also abbreviated as simply FKBP.

The PPIase activity is activity for catalyzing a cis-trans isomerization reaction of peptide bond at the N-terminal side of a proline residue in the protein. The molecular chaperone activity is activity of re-folding a denatured protein into an original normal type, or activity of inhibiting irreversible aggregation of a denatured protein.

The FKBP family protein has action of promoting folding speed of polypeptide based on the PPIase activity and action of suppressing insolubilization by interaction with hydrophobic peptide based on the molecular chaperone activity. The interaction with the hydrophobic peptide sequence with the FKBP family protein enables a nascent polypeptide to be free from degradation by protease.

An origin of the FKBP family protein used in the present invention is not particularly limited. For example, the FKBP family protein derived from prokaryote or archaea (archaebacteria) is used.

The FKBP family protein is generally classified into, depending on the molecular weight, a short type having a molecular weight of 20,000 or less, specifically, a molecular weight of about 16,000 to 18,000, and a long type having a molecular weight of about 26,000 to 33,000. In the present invention, the short type and the long type may be used. The short type is preferably used because the short type has higher molecular chaperone activity. That is to say, in the present invention, a FKBP family protein having a molecular weight of 20,000 or less is preferably used.

Furthermore, examples of FKBP family proteins other than those from archaea are classified mainly into a trigger factor type (Huang, Protein Sci. 9, 1254-, 2000), an FkpA type (Arie, Mol. Microbiol. 39, 199-, 2001), an FKBP52 type (Bose, Science 274, 1715-, 1996), and the like. Any types may be employed in the present invention. For example, the trigger factor type FKBP family protein is used. The FKBP family protein of the trigger factor type has been found in genomes of almost all the bacteria.

FKBP family proteins derived from archaea have been studied in detail (see, for example, WO2004/001041 and WO2005/063964). Examples of the short type FKBP family protein derived from archaea include FKBP family proteins derived from Methanococcus thermolithotrophicus, Thermococcus sp. KS-1, Methanococcus jannaschii, and the like (Maruyama, Front.Biosci. 5, 821-, 2000).

Examples of the long type FKBP family protein derived from archaea include FKBP family proteins derived from Pyrococcus horikoshii, Aeropyrum pernix, Sulfolobus solfataricus, Methanococcus jannaschii, Archaeoglobus fulgidus, Methanobacterium autotrophicum, Thermoplasma acidophilum, Halobacterium cutirubrum, and the like (Maruyama, Front.Biosci. 5, 821-, 2000).

As one example, SEQ ID NO: 7 shows a nucleotide sequence of a nucleic acid (DNA) encoding short type FKBP family protein (TcFKBP18) derived from Thermococcus sp. KS-1 and a corresponding amino acid sequence, and SEQ ID NO: 8 shows only the amino acid sequence.

The FKBP family protein used in the present invention may be not only a naturally occurring and isolated FKBP family protein but also a modified product thereof. For example, it may be proteins that are partial fragments of the existing FKBP family protein or may be amino acid substitution variants and having activity as FKBP family protein. The FKBP family protein activity includes PPIase activity and molecular chaperone activity.

As mentioned above, the PPIase activity is activity for catalyzing a cis-trans isomerization reaction of a peptide bond at the N-terminal side of the proline residue in the protein. Evaluation of the PPIase activity is carried out by, for example, a chymotrypsin coupling method (J. Bacteriol. 1998, 180(2): 388-394).

As mentioned above, the molecular chaperone activity means activity of re-folding a denatured protein into an original normal type, or activity of inhibiting irreversible aggregation of a denatured protein. For example, the molecular chaperone activity is evaluated as follows. That is, rhodanese, citrate synthetase, malate dehydrogenase, glucose-6-phosphate dehydrogenase, and the like, are used as model enzymes (Kawata, Bioscience and Industry 56, 593-, 1998), and these are denaturation-treated with a protein denaturing agent such as 6M guanidine hydrochloride. Then, molecular chaperone activity of a test substrate is evaluated based on a rate of regeneration of a denatured protein which is initiated upon dilution of a denaturing agent with a buffer containing the test substance, and a rate of inhibiting aggregation of a denatured protein. Examples of a method of assessing a rate of regeneration of a denatured protein include a method of Horowitz et al. (Horowitz, Methods Mol. Biol., 40, 361-, 1995) when rhodanese is used. Examples of a method of assessing inhibition of aggregation of a denatured protein include a method of Taguchi et al. (Taguchi, J. Biol. Chem., 269, 8529-, 1994).

For example, the FKBP family protein used in the present invention includes at least the following proteins (d-1) to (d-3):
(d-1) a protein consisting of the amino acid sequence of SEQ ID NO: 8,
(d-2) a protein consisting of the amino acid sequence in which 1 to 20 amino acids are deleted, substituted or added in the amino acid sequence of SEQ ID NO: 8, and having activity as the FKBP family protein, and
(d-3) a protein consisting of the amino acid sequence having homology of 90% or more with the amino acid sequence of SEQ ID NO: 8, and having activity as the FKBP family protein.

Note here that the homology of the amino acid sequence in (d-3) is more preferably 92% or more, further more preferably 95% or more, and particularly preferably 98% or more.

The homology of an amino acid sequence is calculated, for example, by using a multiple sequence alignment program such as CulustalW commercially available.

Next, isoprene synthase is described.

As mentioned above, the isoprene synthase (EC 4.2.3.27) has action of converting dimethylallyl diphosphate (DMAPP) as an isomer of isopentenyl diphosphate (IPP) into isoprene. Note here that structural conversion between IPP and DMAPP is catalyzed by isopentenyl diphosphate isomerase. The isopentenyl diphosphate isomerase is present in all organisms.

Hereinafter, the isoprene synthase is also abbreviated as simply IspS.

The isoprene synthase used in the present invention is not particularly limited. For example, isoprene synthase derived from eukaryote such as plant is used. General examples of the isoprene synthase derived from plants include the isoprene synthase derived from *Populus nigra, Stizolobium deeringianum,* and *Pueraria lobata Ohwi.* Other examples include isoprene synthase derived from genus *Salix,* genus *Robinia,* genus *Wisteria,* genus *Triticum,* genus *Morus,* and the like. All of them can be applied to all the present invention.

SEQ ID NO: 1 shows a nucleotide sequence of a nucleic acid (DNA) encoding the isoprene synthase derived from *Populus nigra* (GenBank Accession No.: AM410988.1) and a corresponding amino acid sequence. SEQ ID NO: 2 shows only the amino acid sequence.

Synthase derived from other than plants includes isoprene synthase derived from prokaryote. Examples thereof include isoprene synthase derived from *Bacillus subtilis* (Sivy TL. et al., Biochem. Biophys. Res. Commu. 2002, 294(1), 71-5).

Also the isoprene synthase used in the present invention may be not only a naturally occurring and isolated isoprene synthase but also a modified product thereof. For example, it may be proteins that are partial fragments of the existing isoprene synthase or may be amino acid substitution variants and having activity as isoprene synthase.

For example, the isoprene synthase used in the present invention includes at least the following (a-1) to (a-3):
(a-1) a protein consisting of the amino acid sequence of SEQ ID NO: 2,
(a-2) a protein consisting of the amino acid sequence in which 1 to 20 amino acids are deleted, substituted or added in the amino acid sequence of SEQ ID NO: 2, and having isoprene synthase activity, and
(a-3) a protein consisting of an amino acid sequence having homology of 90% or more with the amino acid sequence of SEQ ID NO: 2, and having isoprene synthase activity.

Note here that the homology of the amino acid sequence in (a-3) is more preferably 92% or more, further more preferably 95% or more, and particularly preferably 98% or more.

Next, cyclic terpene synthase is described.

As mentioned above, the cyclic terpene synthase is an enzyme for producing cyclic terpene using linear terpene as a substrate. Examples of the cyclic terpene synthase include cyclic monoterpene synthase, cyclic sesquiterpene synthase, cyclic diterpene synthase.

The cyclic monoterpene synthase has action of cyclizing geranyl diphosphate (GPP) to be converted into cyclic monoterpene.

Specific examples of the cyclic monoterpene synthase include phellandrene synthase. For example, β-phellandrene synthase that is one type of the phellandrene synthase has action of converting geranyl diphosphate (GPP) or neryl diphosphate (NPP) into β-phellandrene (Patent Document 5).

Specific examples of the β-phellandrene synthase and the nucleic acid encoding thereof includes β-phellandrene synthase derived from *Solanum lycopersicum* (GenBank Accession No.: FJ797957; Schilmiller, A. L., et al., Proc Natl Acad Sci U S A., 2009, 106, 10865-70.), β-phellandrene synthase derived from *Lavandula angustifolia* (GenBank Accession No.: HQ404305; Demissie, Z. A., et al., Planta, 2011,. 233, 685-96), and the like.

SEQ ID NO: 3 shows a nucleotide sequence of a nucleic acid (DNA) encoding the β-phellandrene synthase derived from *Lavandula angustifolia* and a corresponding amino acid sequence. SEQ ID NO: 4 shows only the amino acid sequence. DNA having the nucleotide sequence set forth in SEQ ID NO: 3 is an example of a nucleic acid encoding the β-phellandrene synthase (cyclic monoterpene synthase).

The cyclic sesquiterpene synthase has action of cyclizing farnesyl diphosphate (FPP) and converting thereof into cyclic sesquiterpene. Examples of the cyclic sesquiterpene synthase include trichodiene synthase.

SEQ ID NO: 5 shows a nucleotide sequence of a nucleic acid (DNA) encoding the trichodiene synthase derived from *Fusarium poae* and a corresponding amino acid sequence. SEQ ID NO: 6 shows only the amino acid sequence. DNA having the nucleotide sequence set forth in SEQ ID NO: 5 is an example of a nucleic acid encoding the trichodiene synthase (cyclic sesquiterpene synthase).

The cyclic diterpene synthase has action of cyclizing geranylgeranyl diphosphate (GGPP) to be converted into cyclic diterpene.

The cyclic terpene synthase used in the present invention may not only a naturally occurring and isolated cyclic terpene synthase but also a modified product thereof. For example, the cyclic terpene synthase may be a protein that is a partial fragment or an amino acid substituted variant of existing cyclic terpene synthase and that has cyclic terpene synthase activity.

For example, the cyclic monoterpene synthase used in the present invention includes at least the following (b-1) to (b-3):
(b-1) a protein consisting of the amino acid sequence of SEQ ID NO: 4,
(b-2) a protein consisting of the amino acid sequence in which 1 to 20 amino acids are deleted, substituted or added in the amino acid sequence of SEQ ID NO: 4, and having β-phellandrene synthase activity, and
(b-3) a protein consisting of an amino acid sequence having homology of 90% or more with the amino acid sequence of SEQ ID NO: 4, and having β-phellandrene synthase activity.
Note here that the homology of the amino acid sequence in (b-3) is more preferably 92% or more, further more preferably 95% or more, and particularly preferably 98% or more.

For example, the cyclic sesquiterpene synthase used in the present invention includes at least the following (c-1) to (c-3):
(c-1) a protein consisting of the amino acid sequence of SEQ ID NO: 6,
(c-2) a protein consisting of the amino acid sequence in which 1 to 20 amino acids are deleted, substituted or added in the amino acid sequence of SEQ ID NO: 6, and having trichodiene synthase activity, and
(c-3) a protein consisting of an amino acid sequence having homology of 90% or more with the amino acid sequence of SEQ ID NO: 6, and having trichodiene synthase activity.

Note here that the homology of the amino acid sequence in (c-3) is more preferably 92% or more, further more preferably 95% or more, and particularly preferably 98% or more.

In a fusion protein encoded by the nucleic acid of the present invention, the link direction of the first protein (isoprene synthase or cyclic terpene synthase) and the FKBP family protein is not particularly limited. That is to say, the FKBP family protein may be linked to the N-terminal side of the first protein, or the FKBP family protein may be linked to the C-terminal side of the first protein. Furthermore, the first protein and the FKBP family protein may be linked to each other directly, or may be linked to each other via a peptide linker or the like. As the peptide linker, for example, a peptide linker that includes a structurally flexible amino acid sequence consisting of about 10 to 50 amino acids is used. For example, a peptide linker consisting of repeating structure of amino acid sequence of four glycines and one serine is used.

The fusion protein may further include a secretion signal. When the secretion signal is provided, the fusion protein can be secreted when it is expressed in a host cell.

The present invention encompasses a fusion protein encoded by the above-mentioned nucleic acid, that is, a fusion protein in which the first protein (isoprene synthase or cyclic terpene synthase) and the FKBP family protein are linked to each other.

The present invention encompasses a recombinant cell that is a bacterium, includes a nucleic acid encoding the above-mentioned fusion protein, and expresses the fusion protein.

Furthermore, the present invention encompasses a recombinant cell that is a bacterium, includes a first nucleic acid encoding a first protein (isoprene synthase or cyclic terpene synthase) and a second nucleic acid encoding a FKBP family protein, and expresses the isoprene synthase or cyclic terpene synthase and the FKBP family protein.

In addition, the present invention encompasses a recombinant cell that is a bacterium, includes a first nucleic acid encoding isoprene synthase and a second nucleic acid encoding a FKBP family protein, and expresses the isoprene synthase and the FKBP family protein.

In any recombinant cells, isoprene synthase or cyclic terpene synthase is stabilized by the presence of adjacent or coexistent FKBP family protein.

A recombinant cell of the present invention is obtained, for example, by introducing the nucleic acid into a host cell such as a bacterium.

In general, prokaryotes such as bacteria have a capability of synthesizing isopentenyl diphosphate (IPP) by a non-mevalonate pathway (MEP pathway). Furthermore, as mentioned above, the isopentenyl diphosphate isomerase is present in all organisms. Therefore, the recombinant cell of the present invention converts DMAPP converted from IPP into isoprene. That is to say, the recombinant cell of the present invention produces isoprene.

Furthermore, the recombinant cell of the present invention converts geranyl diphosphate (GPP), farnesyl diphosphate (FPP), geranylgeranyl diphosphate (GGPP), and the like, having IPP as a precursor, into cyclic terpene. That is to say, the recombinant cell of the present invention produces cyclic terpene.

The host cell is not particularly limited as long as it is bacteria. For example, bacteria used as a host for allowing a foreign gene to express, for example, *Escherichia coli* and *Bacillus subtilis* are applied also in the present invention.

As one embodiment, bacteria having a capability of assimilating a C1 compound are used as a host cell. For example, bacteria having a capability of assimilating carbon monoxide, carbon dioxide, methane, methanol, methyl amine, formic acid, formamide, and the like, are used as a host cell. Thus, a recombinant cell having a capability of assimilating a C1 compound such as carbon monoxide, carbon dioxide, methane, methanol, methyl amine, formic acid, and formamide is obtained.

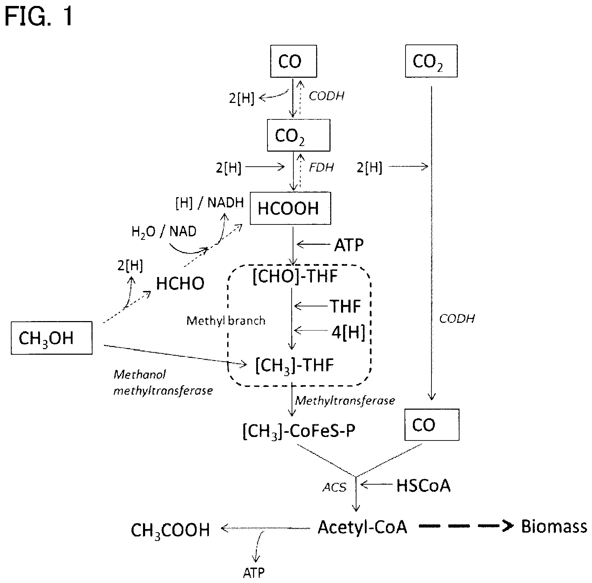

In one embodiment, bacteria having a capability of assimilating carbon monoxide or carbon dioxide are used as a host cell. This makes it possible to obtain a recombinant cell having a capability of assimilating carbon monoxide or carbon dioxide. Specific examples of the host cell include an anaerobic prokaryotic cell. In particular, examples of the host cell include an anaerobic prokaryotic cell having a function of synthesizing acetyl CoA from methyl tetrahydrofolate, carbon monoxide, and CoA. Furthermore, an anaerobic prokaryotic cell further including carbon monoxide dehydrogenase (EC 1.2.99.2) is preferable. In detail, the preferable host cell is an anaerobic prokaryotic cell, which grows mainly by carbon monoxide metabolism, that is, a function of generating carbon dioxide and proton from carbon monoxide and water by the action of the carbon monoxide dehydrogenase. Examples of such an anaerobic prokaryotic cell includes an anaerobic prokaryotic cell having an acetyl CoA pathway (Wood-Ljungdahl pathway) and a methanol pathway shown in Fig. 1.

Use of such an anaerobic prokaryotic cell as a host cell makes it possible to allow a recombinant cell to produce isoprene or cyclic terpene using gas such as carbon monoxide or carbon dioxide as a carbon source.

Examples of the anaerobic prokaryotic cell include a *Clostridium* bacterium or a *Moorella* bacterium, for example, *Clostridium ljungdahlii, Clostridium autoethanogenumn, Clostridium carboxidivorans, Clostridium ragsdalei* (Kopke M. et al., Appl. Environ. Microbiol. 2011, 77(15), 5467-5475), *Moorella thermoacetica* (same as *Clostridium thermoaceticum*) (Pierce EG. Et al., Environ. Microbiol. 2008, 10, 2550-2573). In particular, *Clostridium* bacteria are preferable as the host cell in the recombinant cell of the present invention because their host-vector systems and culture methods have been established. Note here that the five species of *Clostridium* bacteria or *Moorella* bacteria mentioned above are known as representative examples of syngas assimilating microorganisms.

Besides, prokaryotic cells such as *Carboxydocella sporoducens sp. Nov., Rhodopseudomonas gelatinosa, Eubacterium limosum, Butyribacterium methylotrophicum,* and the like, are used as the host cell.

A basic configuration of a technique for allowing a recombinant cell to produce isoprene from syngas is described in the above mentioned Patent Document 1.

As one embodiment, a methanol assimilating bacterium as one type of methylotroph is used as the host cell. This makes it possible to obtain a recombinant cell having methanol assimilating ability.

A methylotroph is a general name for a C1 compound assimilating microorganism that uses a carbon compound that does not have a C-C bond in the molecule, for example, methane, methanol, methylamine, dimethylamine, trimethylamine, or the like, as a sole carbon source or an energy source. All of the microorganisms called methanotroph, methane-oxidizing bacteria, methanol assimilating bacteria, methanol assimilating yeast, methanol assimilating microorganisms belong to methylotrophs. Methanotroph converts methane into methanol by the action of methane monooxygenase, and dissimilates methanol by the same metabolism as in methyltroph.

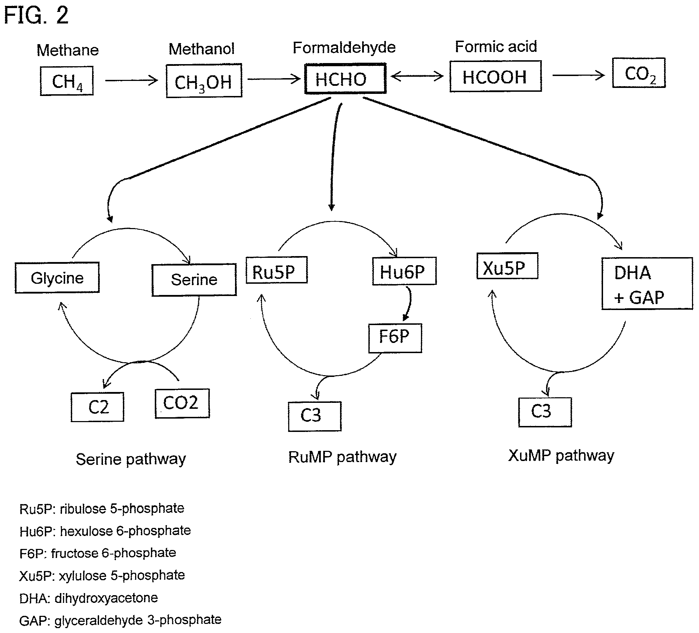

Central metabolism of methylotroph is a reaction of converting methanol into formaldehyde and then converting formaldehyde into an organic compound having a C-C bond. As a carbon assimilation metabolism pathway via formaldehyde, a serine pathway, a ribulose monophosphate pathway (RuMP pathway), and a xylulose monophosphate pathway (XuMP pathway) shown in Fig. 2 are known. Methanol assimilating bacteria used in the present invention have a serine pathway or a RuMP pathway.

Methylotroph has a function of converting methanol and/or formic acid into formaldehyde and a capability of fixing formaldehyde.

Examples of the methanol assimilating bacteria include methylotroph belonging to the genus *Methylacidphilum,* the genus *Methylosinus,* the genus *Methylocystis,* the genus *Methylobacterium,* the genus *Methylocella,* the genus *Methylococcus,* the genus *Methylomonas,* the genus *Methylobacter,* the genus *Methylobacillus,* the genus *Methylophilus,* the genus *Methylotenera,* the genus *Methylovorus,* the genus *Methylomicrobium,* the genus *Methylophaga,* the genus *Methylophilaceae,* the genus *Methyloversatilis,* the genus *Mycobacterium,* the genus *Arthrobacter,* the genus *Bacillus,* the genus *Beggiatoa,* the genus *Burkholderia,* the genus *Granulibacter,* the genus *Hyphomicrobium,* the genus *Pseudomonas,* the genus *Achromobactor,* the genus *Paracoccus,* the genus *Crenothrix,* the genus *Clonothrix,* the genus *Rhodobacter,* the genus *Rhodocyclaceae,* the genus *Silicibacter,* the genus *Thiomicrospira,* and the genus *Verrucomicrobia.*

Bacteria other than methylotroph can be treated as methanol assimilating bacteria by imparting "a function of converting methanol and/or formic acid into formaldehyde" and "formaldehyde fixing ability", which are characteristics of the methylotroph.

A basic configuration of a technique for allowing a recombinant cell as methylotroph to produce isoprene from methanol or methane is described in the above mentioned Patent Document 2.

The method of introducing a gene into the host cell (bacterium) may be selected appropriately depending on the type of the host cell and the like. For example, a method for introducing a target gene by a self-replicating plasmid (vector) or a genome introduction method is used.

For example, an self-replicating plasmid (vector) capable of being introduced into the host cell and capable of expressing the incorporated nucleic acid is used. A vector that autonomously replicates or is incorporated in chromosome in the host cell, and contains a promoter at the position allowing transcription of the inserted nucleic acid (DNA) may be used. For example, it is preferred to construct in the host cell a series of structures including a promoter, a ribosome binding sequence, the above nucleic acid (DNA) and a transcription termination sequence by using the vector.

For introducing a plurality of types of nucleic acids into the host cell by using a vector, the nucleic acids may be incorporated in one vector, or incorporated in individual vectors. When a plurality of types of nucleic acids are incorporated in one vector, these nucleic acids may be expressed under a common promoter for these nucleic acids, or expressed under individual promoters. Examples of introducing a plurality of types of nucleic acids include an embodiment of introducing a nucleic acid encoding the FKBP family protein and a nucleic acid encoding isoprene synthase or cyclic terpene synthase (co-expression).

Examples of the self-replicating vectors to be used when the host is *Escherichia coli* include commercially available pET (available from Novagen), pBAD (available from Life technologies), pGEM (available from Promega), and the like. Examples of vectors when the host is a methanol assimilating bacterium include pAYC32 (Chistoserdov AY., et al., Plasmid 1986, 16, 161-167), pRP301 (Lane M., et al., Arch. Microbiol. 1986, 144(1), 29-34), pBBR1, pBHR1 (Antoine R. et al., Molecular Microbiology 1992, 6, 1785-1799), pCM80 (Marx CJ. et al., Microbiology 2001, 147, 2065-2075), and the like. Examples of vectors when the host is *Clostridium* bacterium include pJIR (Brandshaw M., et al., Plasmid 40 (3), 233-237), pIMP1 (Mermelstein LD et al., Bioltechnology 1992, 10, 190-195), pMTL (Ng, YK. Et al., PLoS One 2013, 8 (2), e56051), pMVTcatMCS47 (Berzin, V. et al., Appl. Biochem. Biotechnol. 2012, 167, 338-347), and the like. These self-replicating vectors are introduced into a host cell by electroporation, a joining method, a chemical treatment method, and the like.

On the other hand, the genome introduction method is carried out using techniques such as homologous recombination, a transposon method (Martinerz-Garcia E. et al., BMC Microbiol. 11:38), an integrase method (Miyazaki, R. et al., Appl. Environ. Microbiol. 2014, 79 (14), 4440-4447), a Cre/loxP method (Bertram, R. et al., J. Mol. Microbiol. Biotechnol. 2009, 17 (3), 136-145), a Flp/FRT method (Al-Hinai, MA. et al., Appl. Environ. Microbiol. 2012, 78 (22), 8112-8121), and the like.

From the viewpoint of improving the production ability of isoprene or cyclic terpene, other nucleic acids may be futher introduced into the recombinant cell. For example, a nucleic acid encoding enzyme (enzyme group) acting on a mevalonate pathway (MVA pathway) or non-mevalonate pathway (MEP pathway) as a synthesis pathway of isopentenyl diphosphate (IPP) is introduced. Thus, synthesis ability of IPP as a supply source of DMAPP is enhanced, resulting in enhancing synthesis ability of isoprene or cyclic terpene.

The method for culturing the recombinant cell of the present invention is not particularly limited. The recombinant cell may be cultured in a medium that allows the recombinant cell to proliferate. For example, when the host is heterotrophic microorganism such as *Escherichia coli* and *Bacillus subtilis,* carbon sources such as glucose and saccharose are used. When the host is methanol assimilating bacteria, a medium containing 0.1 to 5.0% (v/v) methanol is preferably used. When the host is syngas-assimilating bacteria, the followings can be used as carbon sources and energy sources.

CO

CO/H₂

CO₂/H₂

CO/CO₂/H₂

CO/HCOOH

CO₂/HCOOH

CO/CH₃OH

CO₂/CH₃OH

CO/H₂/HCOOH

CO₂/H₂/HCOOH

CO/H₂/CH₃OH

CO₂/H₂/CH₃OH

CO/CO₂/H₂/HCOOH

CO/CO₂/H₂/CH₃OH

CH₃OH/H₂

HCOOH/H₂

Furthermore, even when the host is methanol-assimilating bacteria or syngas-assimilating bacteria, a sugar carbon source such as glucose and saccharose may be added in culture if necessary. Furthermore, the culture may be carried out by any methods such as a batch culture method, a fed-batch culture method, or a continuous culture method. In the fed-batch culture or the continuous culture, the culture solution is subjected to semipermeable membrane circulation, so that culture can be carried out with the cell density enhanced.

The present invention encompasses a method for producing isoprene or cyclic terpene by bringing gas that contains carbon dioxide and hydrogen into contact with the above-mentioned recombinant cell, and allowing the recombinant cell to produce isoprene or cyclic terpene from carbon dioxide. Furthermore, the present invention encompasses a method for producing isoprene or cyclic terpene by bringing gas that contains carbon monoxide, carbon dioxide and hydrogen into contact with the above-mentioned recombinant cell, and allowing the recombinant cell to produce isoprene or cyclic terpene from the carbon monoxide and carbon dioxide.

For example, a recombinant cell using an anaerobic prokaryotic cell having a function of synthesizing acetyl CoA from methyl tetrahydrofolate, carbon monoxide, and CoA mentioned above as a host cell is brought into contact with the above-mentioned gas, and isoprene or cyclic terpene is produced from the above-mentioned gas.

As one example, the recombinant cell is cultured by using the above-mentioned gas as a carbon source, and isoprene or cyclic terpene is isolated from the cultured product.

Besides, isoprene or cyclic terpene is produced by bringing the above-mentioned gas into contact with the recombinant cell regardless of whether or not cell proliferation occurs. For example, the above-mentioned gas is continuously fed to the fixed recombinant cell, so that isoprene or cyclic terpene can be produced continuously.

The isoprene or cyclic terpene produced by a recombinant cell is collected from, for example, an extracellular vapor phase. Examples of the collecting method may include a solid phase adsorption process and a solvent absorption process.

Note here that when the recombinant cell of the present invention is cultured exclusively for cell proliferation, or obtaining a fusion protein itself, rather than for production of isoprene or cyclic terpene isoprene, it is not necessary to use the above-mentioned gas as a carbon source. For example, the recombinant cell may be cultured using other carbon sources such as saccharides or glycerin as mentioned above.

In the following, the present invention will be described more specifically by way of examples. However, the present invention is not limited to these examples.

### EXAMPLE

### [Example 1]

### Expression of FKBP-IspS fusion protein in Escherichia coli BL21 strain (Lon⁻, OmpT) and isoprene production

An artificial synthetic gene (DNA) of SEQ ID NO: 9 was prepared. The artificial synthetic gene includes a gene encoding a fusion protein consisting of FKBP derived from *Thermococcus* and isoprene synthase (IspS) derived from *Populus nigra,* and a gene encoding isopentenyl diphosphate isomerase (IDI) derived from actinomycete. Furthermore, the artificial synthetic gene is designed to have a tag sequence composed of six histidine residues at the C-terminal of the FKBP-IspS fusion protein. The artificial synthetic gene was introduced into NdeI and BamHI cleavage sites of a pET-3a vector to construct an expression vector pTFKIS. pTFKIS was introduced into an *Escherichia coli* BL21 strain (*Lon⁻, OmpT)* to obtain a recombinant.

The recombinant was cultured at 30°C in a 2×YT medium containing 100 µg/mL ampicillin. After 16 hours from the start of culture, 5 mL of culture solution was transferred to TORAST HS 20ml vial (manufactured by SHIMADZU CORPORATION) sealed by a septum cap, IPTG was added thereto at 0.1 mM, and further continued to be cultured at 30°C for 20 hours. After the culture, headspace sampling measurement using GCMS QP2010 ultra (SHIMADZU CORPORATION) was carried out. As a GC column, ZB-624 (manufactured by phenomenex: membrane thickness: 1.40 µm/ length: 30.0 m/ inner diameter: 0.25 mm) was used. An amount of isoprene production per dry cell weight (g) was calculated. Culture was carried out three times in total, and the mean value thereof was employed (N = 3).

On the other hand, after the completion of culture, the cells were collected, and subjected to ultrasonic treatment to obtain a cell disruption solution. The cell disruption solution was centrifuged and separated into a supernatant (a soluble fraction) and a precipitated fraction. Both these fractions were subjected to electrophoresis, and expression of FKBP-IspS fusion protein was assessed by Western blotting using anti-6 His-tag antibody (manufactured by GE Healthcare). The evaluation of the expression level was carried out by evaluating the color intensity of antibody staining in six stages of 0 to 5.

As a control, the same experiment was carried out with respect to *Escherichia coli* BL21 strain (*Lon⁻*, *OmpT⁻*) in which only a pET3a vector had been introduced.

### [Comparative Example 1]

The same experiment was carried out as in Example 1 except that IspS was singly used instead of using a FKBP-IspS fusion protein.

An artificial synthetic gene (DNA) of SEQ ID NO: 10 was prepared. The artificial synthetic gene includes a gene encoding IspS derived from *Populus nigra,* and a gene encoding isopentenyl diphosphate isomerase (IDI) derived from actinomycete, but it does not include an FKBP gene. Furthermore, the artificial synthetic gene is designed to have a tag sequence having six histidine residues at the C-terminal of IspS. The artificial synthetic gene was introduced into NdeI and BamHI cleavage sites of pET-3a vector to construct an expression vector pTIS. pTIS was introduced into an *Escherichia coli* BL21 strain (*Lon⁻*, *OmpT*) to obtain a recombinant.

The recombinant was cultured in the same manner as in Example 1. Furthermore, headspace sampling measurement was carried out in the same manner as in Example 1. Furthermore, the expression of IspS was assessed by Western blotting.

Results are shown in Table 1. That is to say, an amount of isoprene production by an *Escherichia coli* recombinant (Example 1) into which a gene encoding a fusion protein of FKBP and IspS had been introduced was 54.5 mg per gram of dry cell, which was significantly higher than an amount of isoprene production (7.4 mg per gram of dry cell) by an *Escherichia coli* recombinant into which an IspS gene as a non-fused gene had been introduced (Comparative Example 1).

Furthermore, in the expression of IspS (color intensity in Western blotting), in the recombinant of Example 1, a sufficient amount of IspS was detected in the soluble fraction, and only a slight amount of IspS was detected in the precipitated fraction. This showed that when IspS was fused with FKBP, the expression level of the active IspS holding a normal three-dimensional structure in the soluble fraction was increased, thus increasing the isoprene production. On the other hand, in the recombinant of Comparative Example 1, IspS was detected mainly in the precipitated fraction, only a small amount of IspS was detected in the soluble fraction.

As mentioned above, use of *Escherichia coli* recombinant into which a gene encoding a fusion protein of FKBP and IspS had been introduced was able to significantly improve the amount of isoprene production.
[Table 1]

**TABLE 1**

| Vector | Introduced gene | Isoprene production (mg/DCW^{*}) | Color intensity in Western blotting | |
|---|---|---|---|---|
| pTFKIS (Example 1) | FKBP-IspS-IDI | 54.5 | Soluble fraction | 5 |
| | | | Precipitated fraction | 1 |
| pTIS (Comparative Example 1) | IspS-IDI | 7.4 | Soluble fraction | 2 |
| | | | Precipitated fraction | 4 |
| pET3a | - | Not detected | Soluble fraction | 0 |
| | | | Precipitated fraction | 0 |

| | | | | |
|---|---|---|---|---|
| *DCW: Dry cell weight (g) | | | | |

### [Example 2]

### Expression of FKBP-IspS fusion protein in syngas-assimilating bacterium Clostridium ljungdahlii and isoprene production

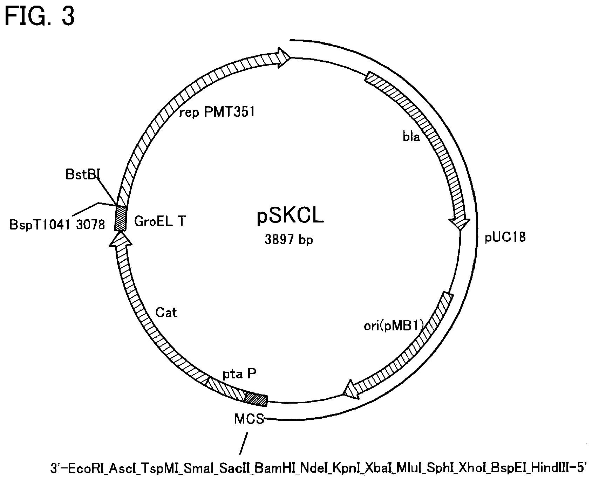

An artificial synthetic gene (DNA) of SEQ ID NO: 11 was prepared. As in Example 1, the artificial synthetic gene includes a gene encoding a fusion protein consisting of FKBP derived from *Thermococcus* and IspS derived from *Populus nigra,* and a gene encoding IDI derived from actinomycete, and designed to induce expression of these genes by a pta (Phosphotransacetylase) promoter derived from C. *ljungdahlii.* Furthermore, the artificial synthetic gene is designed to have a tag sequence composed of six histidine residues at the C-terminal of the FKBP-IspS fusion protein. The artificial synthetic gene was introduced into TspMI and BspEI cleavage sites of a pSKCL vector (Fig. 3, SEQ ID NO: 13) to construct an expression vector pSCLFKIS. pSCLFKIS was introduced into a C. *ljungdahlii* (DSM13528) strain to obtain a recombinant.

The recombinant was cultured at 37°C in 5 mL of PETC 1754 medium (ATCC: American Type Culture Collection) containing 30 µg/mL chloramphenicol in TORAST HS 20ml vial (manufactured by SHIMADZU CORPORATION) sealed by a septum cap. At the time when the OD600 value of the culture solution exceeded 1.0, an isoprene amount in a vapor phase was measured in the same manner as in Example 1. Culture was carried out three times in total, and the mean value thereof was employed (N = 3).

Furthermore, after culture, the cells were collected, and subjected to French press to obtain a cell disruption solution. The cell disruption solution was centrifuged and separated into a supernatant (a soluble fraction) and a precipitated fraction. Both the fractions were subjected to Western blotting in the same manner as in Example 1 to assess the expression of IspS.

As a control, the same experiment was carried out also with respect to C. *ljungdahlii* into which only a pSKCL vector had been introduced.

### [Comparative Example 2]

The same experiment was carried out as in Example 2 except that IspS was singly used instead of using FKBP-IspS fusion protein.

An artificial synthetic gene (DNA) of SEQ ID NO: 12 was prepared. The artificial synthetic gene includes a gene encoding IspS derived from *Populus nigra,* and a gene encoding isopentenyl diphosphate isomerase (IDI) derived from actinomycete, but it does not include an FKBP gene. Furthermore, the artificial synthetic gene is designed to induce expression of these genes by a pta promoter derived from C. *ljungdahlii.* Furthermore, the artificial synthetic gene is designed to have a tag sequence having six histidine residues at the C-terminal of FKBP-IspS fusion protein. The artificial synthetic gene was introduced into TspMI and BspEI cleavage sites of pSKCL vector (Fig. 3) to construct an expression vector pSCLIS. pSCLIS was introduced into a C. *ljungdahlii* (DSM13528) strain to obtain a recombinant.

The recombinant was cultured in the same manner as in Example 2. Furthermore, headspace sampling measurement was carried out in the same manner as in Example 2. Furthermore, the expression of IspS was assessed by Western blotting.

Results are shown in Table 2. That is to say, an amount of isoprene production by a *C. ljungdahlii* recombinant (Example 2) into which a gene encoding a fusion protein of FKBP and IspS had been introduced was 2963 µg per gram of dry cell, which was significantly higher than an amount of isoprene production (0.50 µg per gram of dry cell) by a C. *ljungdahlii recombinant* into which an IspS gene as a non-fused gene had been introduced (Comparative Example 2).

Furthermore, in the expression of IspS (color intensity in Western blotting), in the recombinant of Example 2, a sufficient amount of IspS was detected in the soluble fraction, and only a slight amount of IspS was detected in the precipitated fraction. On the contrary, in the recombinant of Comparative Example 2, IspS was hardly detected, and it was slightly observed in the precipitated fraction. This was thought to be because in C. *ljungdahlii,* non-fused IspS was not able to form a normal three-dimensional structure, and almost all was degraded by protease.

As mentioned above, use of C. *ljungdahlii* recombinant into which a gene encoding a fusion protein FKBP and IspS had been introduced made it possible to remarkably improve the amount of isoprene production.
[Table 2]

**TABLE 2**

| Vector | Introduced gene | Isoprene production (mg/DCW^{*}) | Color intensity in Western blotting | |
|---|---|---|---|---|
| pSCLFKIS (Example 2) | FKBP-IspS-IDI | 2963 | Soluble fraction | 4 |
| | | | Precipitated fraction | 1 |
| pSC LIS (Comparative Example 2) | IspS-IDI | 0.50 | Soluble fraction | 0 |
| | | | Precipitated fraction | 1 |
| pSKCL | - | 0.36 | Soluble fraction | 0 |
| | | | Precipitated fraction | 0 |

| | | | | |
|---|---|---|---|---|
| *DCW: Dry cell weight (g) | | | | |

### Example 3

### Expression of FKBP-β-phellandrene fusion protein in methylotroph Methylobacterium extorquens and production of β-phellandrene

In this Example, recombinant *Methylobacterium extorquens* for producing β-phellandrene as one type of cyclic monoterpene was prepared. Note here that *M. extorquens* holds an enzyme for synthesizing geranyl diphosphate (GPP), which is a substrate of β-phellandrene synthase, from isopentenyl diphosphate (IPP). Therefore, it was thought that when a β-phellandrene synthase gene was introduced into this strain, and the gene was allowed to express, thus enabling β-phellandrene to be synthesized from methanol.

An artificial synthetic gene (DNA) of SEQ ID NO: 14 was prepared. The artificial synthetic gene includes a gene encoding a fusion protein consisting of FKBP derived from *Thermococcus* and β-phellandrene synthase (bPHS) derived from *Lavandula angustifolia,* and designed to induce expression of the gene by the pta promoter used in Example 2. The artificial synthetic gene was introduced into a BamHI/KpnI site of pCM80 (Marx CJ. et al., Microbiology 2001, 147, 2065-2075) set forth in SEQ ID NO: 15 as a broad host range vector to prepare pC80FkPHS. The expression vector pCM80FkPHS was introduced into *M*. *extorquens* by electroporation to obtain a ME-FkPHS strain.

The ME-FkPHS strain was cultured aerobically at 30°C in 20 mL of synthetic medium containing methanol as a sole carbon source (1 L of the synthetic medium contains 18 g of H₃PO₄, 14.28 g of K₂SO₄, 3.9 g of KOH, 0.9 g of CaSO₄•2H₂O, 11.7 g of MgSO₄•7H₂O, 8.4 mg of CuSO₄•5H₂O, 1.1 mg of KI, 4.2 mg of MnSO₄H₂O, 0.3 mg of NaMoO₄•2H₂O, 0.03 mg of H₃BO₃, 0.7 mg of CoCl₂•6H₂O, 28 mg of ZnSO₄•7H₂O, 91 mg of FeSO₄•7H₂O, 0.28 mg of biotin, 5 mL of methanol, and 10 mg of tetracycline). At the time when the OD600 value of the culture solution was 1.0 to 1.2, cells were collected. A part of the collected cells was cultured at 30°C in 5 mL of the above-mentioned synthetic medium containing 20 µg/mL tetracycline in TORAST HS 20ml vial (manufactured by SHIMADZU CORPORATION) sealed by a septum cap. At the time when the OD600 value of the culture solution exceeded 1.0, a β-phellandrene amount in a vapor phase was measured in the same manner as in Example 1. Culture was carried out three times in total, and the mean value thereof was employed (N = 3).

As a control, the same experiment was carried out also in *M. extorquens* into which only a pCM80 vector had been introduced.

As a result, *M. extorquens* into which pCM80 had been introduced did not produce β-phellandrene at all, but the ME-FkPHS strain produced 7.3 mg per gram of dry cell of β-phellandrene.

### [Comparative Example 3]

The same experiment was carried out as in Example 3 except that bPHS was singly used instead of a FKBP-bPHS fusion protein.

An artificial synthetic gene of SEQ ID NO: 16 was prepared. The artificial synthetic gene includes a gene encoding only β-phellandrene synthase (bPHS) derived from *Lavandula angustifolia.* The artificial synthetic gene is designed to induce expression of the gene by the pta promoter used in Example 3. The artificial synthetic gene was introduced into a BamHI/KpnI site of pCM80 used in Example 3 to prepare pC80PHS. The expression vector pCM80PHS was introduced into *M. extorquens* by electroporation to obtain an ME-PHS strain. Culture was carried out in the same manner as in Example 3.

As a result, an amount of β-phellandrene production by ME-PHS strain was 0.4 mg per gram of dry cell. From the above-mentioned results, the amount of β-phellandrene production was able to be remarkably increased by fusing FKBP and bPHS also in methylotroph.

## Claims

1. A nucleic acid encoding a fusion protein, the fusion protein comprising:
a first protein selected from the group consisting of isoprene synthase and cyclic terpene synthase; and
a FKBP family protein linked to the first protein.

2. The nucleic acid according to claim 1, wherein the first protein is isoprene synthase.

3. The nucleic acid according to claim 2, wherein the isoprene synthase is any one of the following (a-1) to (a-3):
(a-1) a protein consisting of the amino acid sequence of SEQ ID NO: 2,
(a-2) a protein consisting of the amino acid sequence in which 1 to 20 amino acids are deleted, substituted or added in the amino acid sequence of SEQ ID NO: 2, and having isoprene synthase activity, and
(a-3) a protein consisting of an amino acid sequence having homology of 90% or more with the amino acid sequence of SEQ ID NO: 2, and having isoprene synthase activity.

4. The nucleic acid according to claim 1, wherein the first protein is cyclic monoterpene synthase.

5. The nucleic acid according to claim 4, wherein the cyclic monoterpene synthase is phellandrene synthase.

6. The nucleic acid according to claim 4, wherein the cyclic monoterpene synthase is any one of the following (b-1) to (b-3):
(b-1) a protein consisting of the amino acid sequence of SEQ ID NO: 4,
(b-2) a protein consisting of the amino acid sequence in which 1 to 20 amino acids are deleted, substituted or added in the amino acid sequence of SEQ ID NO: 4, and having β-phellandrene synthase activity, and
(b-3) a protein consisting of an amino acid sequence having homology of 90% or more with the amino acid sequence of SEQ ID NO: 4, and having β-phellandrene synthase activity.

7. The nucleic acid according to claim 1, wherein the first protein is cyclic sesquiterpene synthase.

8. The nucleic acid according to claim 7, wherein the cyclic sesquiterpene synthase is any one of the following (c-1) to (c-3):
(c-1) a protein consisting of the amino acid sequence of SEQ ID NO: 6,
(c-2) a protein consisting of the amino acid sequence in which 1 to 20 amino acids are deleted, substituted or added in the amino acid sequence of SEQ ID NO: 6, and having trichodiene synthase activity, and
(c-3) a protein consisting of an amino acid sequence having homology of 90% or more with the amino acid sequence of SEQ ID NO: 6, and having trichodiene synthase activity.

9. The nucleic acid according to claim 1, wherein the first protein is cyclic diterpene synthase.

10. The nucleic acid according to any one of claims 1 to 9, wherein the first protein is derived from prokaryote.

11. The nucleic acid according to any one of claims 1 to 10, wherein the FKBP family protein is derived from prokaryote.

12. The nucleic acid according to any one of claims 1 to 10, wherein the FKBP family protein is derived from archaea.

13. The nucleic acid according to any one of claims 1 to 12, wherein the FKBP family protein has a molecular weight of 20,000 or less.

14. The nucleic acid according to any one of claims 1 to 10, wherein the FKBP family protein is any one of the following (d-1) to (d-3):
(d-1) a protein consisting of the amino acid sequence of SEQ ID NO: 8,
(d-2) a protein consisting of the amino acid sequence in which 1 to 20 amino acids are deleted, substituted or added in the amino acid sequence of SEQ ID NO: 8, and having activity as the FKBP family protein, and
(d-3) a protein consisting of the amino acid sequence having homology of 90% or more with the amino acid sequence of SEQ ID NO: 8, and having activity as the FKBP family protein.

15. The nucleic acid according to any one of claims 1 to 10, wherein the FKBP family protein belongs to a trigger factor.

16. The nucleic acid according to any one of claims 1 to 15, wherein the FKBP family protein is linked to an N-terminal side of the first protein.

17. The nucleic acid according to any one of claims 1 to 16, wherein the fusion protein further comprises a secretion signal sequence.

18. A fusion protein encoded by the nucleic acid according to any one of claims 1 to 17.

19. A recombinant cell being a bacterium, comprising the nucleic acid according to any one of claims 1 to 17, and expressing the fusion protein.

20. A recombinant cell being a bacterium, comprising:
a first nucleic acid encoding a first protein selected from the group consisting of isoprene synthase and cyclic terpene synthase; and
a second nucleic acid encoding a FKBP family protein,
wherein the recombinant cell expresses the isoprene synthase or the cyclic terpene synthase, and expresses the FKBP family protein.

21. The recombinant cell according to claim 20, wherein the first protein is isoprene synthase, and the recombinant cell expresses the isoprene synthase and the FKBP family protein.

22. The recombinant cell according to claim 20, wherein the first protein is cyclic monoterpene synthase.

23. The recombinant cell according to claim 22, wherein the cyclic monoterpene synthase is phellandrene synthase.

24. The recombinant cell according to claim 20, wherein the first protein is cyclic sesquiterpene synthase.

25. The recombinant cell according to claim 20, wherein the first protein is cyclic diterpene synthase.

26. The recombinant cell according to any one of claims 19 to 25, having a capability of assimilating a C1 compound.

27. The recombinant cell according to claim 26, having a capability of assimilating methanol or methane.

28. The recombinant cell according to claim 26, having a capability of assimilating carbon monoxide or carbon dioxide.

29. The recombinant cell according to claim 28, being an anaerobic bacterium.

30. The recombinant cell according to claim 29, being a *Clostridium* bacterium or a *Moorella* bacterium.

31. A method for producing isoprene or cyclic terpene, the method comprising:
bringing gas that includes carbon dioxide and hydrogen into contact with the recombinant cell according to any one of claims 26 to 30; and
allowing the recombinant cell to produce isoprene or cyclic terpene from carbon dioxide.

32. The method according to claim 31, wherein the gas includes carbon monoxide, carbon dioxide and hydrogen, and the method allows the recombinant cell to produce isoprene or cyclic terpene from carbon monoxide and carbon dioxide.

33. The method according to claim 31 or 32, wherein isoprene or cyclic terpene released to outside of the recombinant cell is collected.

34. The method according to claim 33, wherein the isoprene or cyclic terpene is collected by a solid phase adsorption process.

35. The method according to claim 33, wherein the isoprene or cyclic terpene is collected by a solvent absorption process.


SEQUENCE LISTING

<110>  Sekisui Chemical Co., Ltd.

<120>  NUCLEIC ACID, FUSION PROTEIN, RECOMBINED CELL, AND ISOPRENE OR CYCLIC
TERPENE PRODUCTION METHOD

<130>  P0004178-PCT

<150>  JP2015-026156
<151>  2015-02-13

<150>  JP2015-218114
<151>  2015-11-06

<160>  16

<170>  PatentIn version 3.1

<210>  1
<211>  1788
<212>  DNA
<213>  Populus nigra

<220>
<221>  CDS
<222>  (1)..(1788)
<223>

<400>  1
atg gca act gaa tta ttg tgc ttg cac cgt cca atc tca ctg aca cac       48
Met Ala Thr Glu Leu Leu Cys Leu His Arg Pro Ile Ser Leu Thr His
1               5                   10                  15

aaa ttg ttc aga aat ccc ttg cct aaa gtc atc cag gcc act ccc tta       96
Lys Leu Phe Arg Asn Pro Leu Pro Lys Val Ile Gln Ala Thr Pro Leu
            20                  25                  30

act ttg aaa ctc aga tgt tct gta agc aca gaa aac gtc agc ttc aca      144
Thr Leu Lys Leu Arg Cys Ser Val Ser Thr Glu Asn Val Ser Phe Thr
        35                  40                  45

gaa aca gaa aca gaa acc aga agg tct gcc aat tat gaa cca aat agc      192
Glu Thr Glu Thr Glu Thr Arg Arg Ser Ala Asn Tyr Glu Pro Asn Ser
    50                  55                  60

tgg gat tat gat tat ttg ctg tct tcg gac act gac gaa tcg att gaa      240
Trp Asp Tyr Asp Tyr Leu Leu Ser Ser Asp Thr Asp Glu Ser Ile Glu
65                  70                  75                  80

gta tac aaa gac aag gcc aaa aag ctg gag gct gag gtg aga aga gag      288
Val Tyr Lys Asp Lys Ala Lys Lys Leu Glu Ala Glu Val Arg Arg Glu
                85                  90                  95

att aac aat gaa aag gca gag ttt ttg act ctg cct gaa ctg ata gat      336
Ile Asn Asn Glu Lys Ala Glu Phe Leu Thr Leu Pro Glu Leu Ile Asp
            100                 105                 110

aat gtc caa agg tta gga tta ggt tac cgg ttc gag agt gac ata agg      384
Asn Val Gln Arg Leu Gly Leu Gly Tyr Arg Phe Glu Ser Asp Ile Arg
        115                 120                 125

aga gcc ctt gat aga ttt gtt tct tca gga gga ttt gat gct gtt aca      432

Arg Ala Leu Asp Arg Phe Val Ser Ser Gly Gly Phe Asp Ala Val Thr
    130                 135                 140

aaa act agc ctt cat gct act gct ctt agc ttc agg ctt ctc aga cag 480
Lys Thr Ser Leu His Ala Thr Ala Leu Ser Phe Arg Leu Leu Arg Gln
145                 150                 155                 160

cat ggc ttt gag gtc tct caa gaa gcg ttc agc gga ttc aag gat caa 528
His Gly Phe Glu Val Ser Gln Glu Ala Phe Ser Gly Phe Lys Asp Gln
                165                 170                 175

aat ggc aat ttc ttg aaa aac ctt aag gag gac atc aag gca ata cta 576
Asn Gly Asn Phe Leu Lys Asn Leu Lys Glu Asp Ile Lys Ala Ile Leu
            180                 185                 190

agc cta tat gaa gct tca ttt ctt gcc tta gaa gga gaa aat atc ttg 624
Ser Leu Tyr Glu Ala Ser Phe Leu Ala Leu Glu Gly Glu Asn Ile Leu
        195                 200                 205

gat gag gcc aag gtg ttt gca ata tca cat cta aaa gag ctc agc gaa 672
Asp Glu Ala Lys Val Phe Ala Ile Ser His Leu Lys Glu Leu Ser Glu
    210                 215                 220

gaa aag att gga aaa gac ctg gcc gaa cag gtg aat cat gca ttg gag 720
Glu Lys Ile Gly Lys Asp Leu Ala Glu Gln Val Asn His Ala Leu Glu
225                 230                 235                 240

ctt cca ttg cat cga agg acg caa aga cta gaa gct gtt tgg agc att 768
Leu Pro Leu His Arg Arg Thr Gln Arg Leu Glu Ala Val Trp Ser Ile
                245                 250                 255

gaa gca tac cgt aaa aag gaa gat gca gat caa gta ctg cta gaa ctt 816
Glu Ala Tyr Arg Lys Lys Glu Asp Ala Asp Gln Val Leu Leu Glu Leu
            260                 265                 270

gct ata ttg gac tac aac atg att caa tca gta tac caa aga gat ctt 864
Ala Ile Leu Asp Tyr Asn Met Ile Gln Ser Val Tyr Gln Arg Asp Leu
        275                 280                 285

cgc gag aca tca agg tgg tgg agg cgt gtg ggt ctt gca aca aag ttg 912
Arg Glu Thr Ser Arg Trp Trp Arg Arg Val Gly Leu Ala Thr Lys Leu
    290                 295                 300

cat ttt gct aga gac agg tta att gaa agc ttt tac tgg gca gtt gga 960
His Phe Ala Arg Asp Arg Leu Ile Glu Ser Phe Tyr Trp Ala Val Gly
305                 310                 315                 320

gtt gcg ttt gaa cct caa tac agt gat tgc cgt aat tcc gta gca aaa 1008
Val Ala Phe Glu Pro Gln Tyr Ser Asp Cys Arg Asn Ser Val Ala Lys
                325                 330                 335

atg ttt tcg ttt gta aca atc att gat gat atc tat gat gtt tat ggt 1056
Met Phe Ser Phe Val Thr Ile Ile Asp Asp Ile Tyr Asp Val Tyr Gly
            340                 345                 350

act ctg gat gag ttg gag cta ttt aca gat gct gtt gag aga tgg gat 1104
Thr Leu Asp Glu Leu Glu Leu Phe Thr Asp Ala Val Glu Arg Trp Asp
        355                 360                 365

gtt aat gcc atc gat gat ctt ccg gat tat atg aag ctc tgc ttc cta 1152
Val Asn Ala Ile Asp Asp Leu Pro Asp Tyr Met Lys Leu Cys Phe Leu
    370                 375                 380

gct ctc tat aac act atc aat gag ata gct tat gat aat ctg aag gac 1200
Ala Leu Tyr Asn Thr Ile Asn Glu Ile Ala Tyr Asp Asn Leu Lys Asp
385                 390                 395                 400

aag ggg gaa aac att ctt cca tac cta aca aaa gcg tgg gca gat tta 1248
Lys Gly Glu Asn Ile Leu Pro Tyr Leu Thr Lys Ala Trp Ala Asp Leu
                405                 410                 415

tgc aat gca ttc cta caa gaa gca aaa tgg ttg tac aat aag tcc aca 1296
Cys Asn Ala Phe Leu Gln Glu Ala Lys Trp Leu Tyr Asn Lys Ser Thr
            420                 425                 430

cca aca ttt gat gaa tat ttc gga aat gca tgg aaa tca tcc tca ggg 1344
Pro Thr Phe Asp Glu Tyr Phe Gly Asn Ala Trp Lys Ser Ser Ser Gly
        435                 440                 445

cct ctt caa cta gtt ttt gcc tac ttt gcc gtt gtt caa aac atc aag 1392
Pro Leu Gln Leu Val Phe Ala Tyr Phe Ala Val Val Gln Asn Ile Lys
    450                 455                 460

aaa gag gaa att gat aac tta caa aag tat cat gat atc atc agt agg 1440
Lys Glu Glu Ile Asp Asn Leu Gln Lys Tyr His Asp Ile Ile Ser Arg
465                 470                 475                 480

cct tcc cac atc ttt cgt ctt tgc aac gac ttg gct tca gca tcg gct 1488
Pro Ser His Ile Phe Arg Leu Cys Asn Asp Leu Ala Ser Ala Ser Ala
                485                 490                 495

gag ata gcg aga ggt gaa acc gcg aat tct gta tca tgc tac atg cgt 1536
Glu Ile Ala Arg Gly Glu Thr Ala Asn Ser Val Ser Cys Tyr Met Arg
            500                 505                 510

aca aaa ggc att tct gag gaa ctt gct act gaa tcc gta atg aat ttg 1584
Thr Lys Gly Ile Ser Glu Glu Leu Ala Thr Glu Ser Val Met Asn Leu
        515                 520                 525

atc gac gaa acc tgg aaa aag atg aac aaa gaa aag ctt ggt ggc tct 1632
Ile Asp Glu Thr Trp Lys Lys Met Asn Lys Glu Lys Leu Gly Gly Ser
    530                 535                 540

ctg ttt gca aaa cct ttt gtc gaa aca gct att aac ctt gca cga caa 1680
Leu Phe Ala Lys Pro Phe Val Glu Thr Ala Ile Asn Leu Ala Arg Gln
545                 550                 555                 560

tcc cat tgc act tat cac aac gga gat gcg cat act tca cca gat gag 1728
Ser His Cys Thr Tyr His Asn Gly Asp Ala His Thr Ser Pro Asp Glu
                565                 570                 575

ctc act agg aaa cgt gtc ctg tca gta atc aca gag cct att cta ccc 1776
Leu Thr Arg Lys Arg Val Leu Ser Val Ile Thr Glu Pro Ile Leu Pro
            580                 585                 590

ttt gag aga taa 1788
Phe Glu Arg
        595

<210> 2
<211> 595
<212> PRT
<213> Populus nigra

<400> 2

Met Ala Thr Glu Leu Leu Cys Leu His Arg Pro Ile Ser Leu Thr His
1               5                   10                  15

Lys Leu Phe Arg Asn Pro Leu Pro Lys Val Ile Gln Ala Thr Pro Leu
            20                  25                  30

Thr Leu Lys Leu Arg Cys Ser Val Ser Thr Glu Asn Val Ser Phe Thr
        35                  40                  45

Glu Thr Glu Thr Glu Thr Arg Arg Ser Ala Asn Tyr Glu Pro Asn Ser
    50                  55                  60

Trp Asp Tyr Asp Tyr Leu Leu Ser Ser Asp Thr Asp Glu Ser Ile Glu
65                  70                  75                  80

Val Tyr Lys Asp Lys Ala Lys Lys Leu Glu Ala Glu Val Arg Arg Glu
                85                  90                  95

Ile Asn Asn Glu Lys Ala Glu Phe Leu Thr Leu Pro Glu Leu Ile Asp
            100                 105                 110

Asn Val Gln Arg Leu Gly Leu Gly Tyr Arg Phe Glu Ser Asp Ile Arg
        115                 120                 125

Arg Ala Leu Asp Arg Phe Val Ser Ser Gly Gly Phe Asp Ala Val Thr
    130                 135                 140

Lys Thr Ser Leu His Ala Thr Ala Leu Ser Phe Arg Leu Leu Arg Gln
145                 150                 155                 160

His Gly Phe Glu Val Ser Gln Glu Ala Phe Ser Gly Phe Lys Asp Gln
                165                 170                 175

Asn Gly Asn Phe Leu Lys Asn Leu Lys Glu Asp Ile Lys Ala Ile Leu
            180                 185                 190

Ser Leu Tyr Glu Ala Ser Phe Leu Ala Leu Glu Gly Glu Asn Ile Leu
        195                 200                 205

Asp Glu Ala Lys Val Phe Ala Ile Ser His Leu Lys Glu Leu Ser Glu
    210                 215                 220

Glu Lys Ile Gly Lys Asp Leu Ala Glu Gln Val Asn His Ala Leu Glu
225                 230                 235                 240

Leu Pro Leu His Arg Arg Thr Gln Arg Leu Glu Ala Val Trp Ser Ile
                245                 250                 255

Glu Ala Tyr Arg Lys Lys Glu Asp Ala Asp Gln Val Leu Leu Glu Leu
            260                 265                 270

Ala Ile Leu Asp Tyr Asn Met Ile Gln Ser Val Tyr Gln Arg Asp Leu
        275                 280                 285

Arg Glu Thr Ser Arg Trp Trp Arg Arg Val Gly Leu Ala Thr Lys Leu
    290                 295                 300

His Phe Ala Arg Asp Arg Leu Ile Glu Ser Phe Tyr Trp Ala Val Gly
305                 310                 315                 320

Val Ala Phe Glu Pro Gln Tyr Ser Asp Cys Arg Asn Ser Val Ala Lys
                325                 330                 335

Met Phe Ser Phe Val Thr Ile Ile Asp Asp Ile Tyr Asp Val Tyr Gly
            340                 345                 350

Thr Leu Asp Glu Leu Glu Leu Phe Thr Asp Ala Val Glu Arg Trp Asp
        355                 360                 365

Val Asn Ala Ile Asp Asp Leu Pro Asp Tyr Met Lys Leu Cys Phe Leu
    370                 375                 380

Ala Leu Tyr Asn Thr Ile Asn Glu Ile Ala Tyr Asp Asn Leu Lys Asp
385                 390                 395                 400

Lys Gly Glu Asn Ile Leu Pro Tyr Leu Thr Lys Ala Trp Ala Asp Leu
                405                 410                 415

Cys Asn Ala Phe Leu Gln Glu Ala Lys Trp Leu Tyr Asn Lys Ser Thr
            420                 425                 430

Pro Thr Phe Asp Glu Tyr Phe Gly Asn Ala Trp Lys Ser Ser Ser Gly
        435                 440                 445

Pro Leu Gln Leu Val Phe Ala Tyr Phe Ala Val Val Gln Asn Ile Lys
    450                 455                 460

Lys Glu Glu Ile Asp Asn Leu Gln Lys Tyr His Asp Ile Ile Ser Arg
465                 470                 475                 480

Pro Ser His Ile Phe Arg Leu Cys Asn Asp Leu Ala Ser Ala Ser Ala
                485                 490                 495

Glu Ile Ala Arg Gly Glu Thr Ala Asn Ser Val Ser Cys Tyr Met Arg

500                 505                 510

Thr Lys Gly Ile Ser Glu Glu Leu Ala Thr Glu Ser Val Met Asn Leu
        515                 520                 525

Ile Asp Glu Thr Trp Lys Lys Met Asn Lys Glu Lys Leu Gly Gly Ser
    530                 535                 540

Leu Phe Ala Lys Pro Phe Val Glu Thr Ala Ile Asn Leu Ala Arg Gln
545                 550                 555                 560

Ser His Cys Thr Tyr His Asn Gly Asp Ala His Thr Ser Pro Asp Glu
                565                 570                 575

Leu Thr Arg Lys Arg Val Leu Ser Val Ile Thr Glu Pro Ile Leu Pro
            580                 585                 590

Phe Glu Arg
        595

<210>  3
<211>  1746
<212>  DNA
<213>  Lavandula angustifolia

<220>
<221>  CDS
<222>  (1)..(1746)
<223>

<400>  3
atg tct acc att att gcg ata caa gtg ttg ctt cct att cca act act      48
Met Ser Thr Ile Ile Ala Ile Gln Val Leu Leu Pro Ile Pro Thr Thr
1               5                   10                  15

aaa aca tac cct agt cat gac ttg gag aag tcc tct tcg cgg tgt cgc      96
Lys Thr Tyr Pro Ser His Asp Leu Glu Lys Ser Ser Ser Arg Cys Arg
            20                  25                  30

tcc tcc tcc act cct cgc cct aga ctg tgt tgc tcg ttg cag gtg agt      144
Ser Ser Ser Thr Pro Arg Pro Arg Leu Cys Cys Ser Leu Gln Val Ser
        35                  40                  45

gat ccg atc cca acg ggc cgg cga tcc gga ggc tac ccg ccc gcc cta      192
Asp Pro Ile Pro Thr Gly Arg Arg Ser Gly Gly Tyr Pro Pro Ala Leu
    50                  55                  60

tgg gat ttc gac act att caa tcg ctc aac acc gag tat aag gga gag      240
Trp Asp Phe Asp Thr Ile Gln Ser Leu Asn Thr Glu Tyr Lys Gly Glu
65                  70                  75                  80

agg cac atg aga agg gaa gaa gac cta att ggg caa gtt aga gag atg      288
Arg His Met Arg Arg Glu Glu Asp Leu Ile Gly Gln Val Arg Glu Met
                85                  90                  95

ctg gtg cat gaa gta gag gat ccc act cca cag ctg gag ttc att gat      336

Leu Val His Glu Val Glu Asp Pro Thr Pro Gln Leu Glu Phe Ile Asp
            100                 105                 110

gat ttg cat aag ctt ggc ata tct tgc cat ttt gag aat gaa atc ctc 384
Asp Leu His Lys Leu Gly Ile Ser Cys His Phe Glu Asn Glu Ile Leu
        115                 120                 125

caa atc ttg aaa tcc ata tat ctt aat caa aac tac aaa agg gat ttg 432
Gln Ile Leu Lys Ser Ile Tyr Leu Asn Gln Asn Tyr Lys Arg Asp Leu
    130                 135                 140

tac tca aca tct cta gca ttc aga ctc ctc aga caa tat ggc ttc atc 480
Tyr Ser Thr Ser Leu Ala Phe Arg Leu Leu Arg Gln Tyr Gly Phe Ile
145                 150                 155                 160

ctt cca caa gaa gta ttt gat tgt ttc aag aat gag gag ggt acg gat 528
Leu Pro Gln Glu Val Phe Asp Cys Phe Lys Asn Glu Glu Gly Thr Asp
                165                 170                 175

ttc aag cca agc ttc ggc cgt gat atc aaa ggc ttg tta caa ttg tat 576
Phe Lys Pro Ser Phe Gly Arg Asp Ile Lys Gly Leu Leu Gln Leu Tyr
            180                 185                 190

gaa gct tct ttc cta tca aga aaa gga gaa gaa act tta caa cta gca 624
Glu Ala Ser Phe Leu Ser Arg Lys Gly Glu Glu Thr Leu Gln Leu Ala
        195                 200                 205

aga gag ttt gca aca aag att ctg caa aaa gaa gtt gat gag aga gag 672
Arg Glu Phe Ala Thr Lys Ile Leu Gln Lys Glu Val Asp Glu Arg Glu
    210                 215                 220

ttt gca acc aag atg gag ttc cct tct cat tgg acg gtt caa atg ccg 720
Phe Ala Thr Lys Met Glu Phe Pro Ser His Trp Thr Val Gln Met Pro
225                 230                 235                 240

aat gca aga cct ttc atc gat gct tac cgt agg agg ccg gat atg aat 768
Asn Ala Arg Pro Phe Ile Asp Ala Tyr Arg Arg Arg Pro Asp Met Asn
                245                 250                 255

cca gtt gtg ctc gag cta gcc ata ctt gat aca aat ata gtt caa gca 816
Pro Val Val Leu Glu Leu Ala Ile Leu Asp Thr Asn Ile Val Gln Ala
            260                 265                 270

caa ttt caa gaa gaa ctc aaa gag acc tca agg tgg tgg gag agt aca 864
Gln Phe Gln Glu Glu Leu Lys Glu Thr Ser Arg Trp Trp Glu Ser Thr
        275                 280                 285

ggc att gtc caa gag ctt cca ttt gtg agg gat agg att gtg gaa ggc 912
Gly Ile Val Gln Glu Leu Pro Phe Val Arg Asp Arg Ile Val Glu Gly
    290                 295                 300

tac ttt tgg acg att gga gtg act cag aga cgc gag cat gga tac gaa 960
Tyr Phe Trp Thr Ile Gly Val Thr Gln Arg Arg Glu His Gly Tyr Glu
305                 310                 315                 320

aga atc atg acc gca aag gtt att gcc tta gta aca tgt tta gac gac 1008
Arg Ile Met Thr Ala Lys Val Ile Ala Leu Val Thr Cys Leu Asp Asp
                325                 330                 335

ata tac gat gtt tat ggc acg ata gaa gag ctt caa ctt ttc aca agc 1056
Ile Tyr Asp Val Tyr Gly Thr Ile Glu Glu Leu Gln Leu Phe Thr Ser
            340                 345                 350

aca atc caa aga tgg gat ttg gaa tca atg aag caa ctc cct acc tac 1104
Thr Ile Gln Arg Trp Asp Leu Glu Ser Met Lys Gln Leu Pro Thr Tyr
        355                 360                 365

atg caa gta agc ttt ctt gca cta cac aac ttt gta acc gag gtg gct 1152
Met Gln Val Ser Phe Leu Ala Leu His Asn Phe Val Thr Glu Val Ala
    370                 375                 380

tac gat act ctc aag aaa aag ggc tac aac tcc aca cca tat tta aga 1200
Tyr Asp Thr Leu Lys Lys Lys Gly Tyr Asn Ser Thr Pro Tyr Leu Arg
385                 390                 395                 400

aaa acg tgg gtg gat ctt gtt gaa tca tat atc aaa gag gca act tgg 1248
Lys Thr Trp Val Asp Leu Val Glu Ser Tyr Ile Lys Glu Ala Thr Trp
                405                 410                 415

tac tac aac ggt tat aaa cct agt atg caa gaa tac ctt aac aat gca 1296
Tyr Tyr Asn Gly Tyr Lys Pro Ser Met Gln Glu Tyr Leu Asn Asn Ala
            420                 425                 430

tgg ata tca gtc gga agt atg gct ata ctc aac cac ctc ttc ttc cgg 1344
Trp Ile Ser Val Gly Ser Met Ala Ile Leu Asn His Leu Phe Phe Arg
        435                 440                 445

ttc aca aac gag aga atg cat aaa tac cgc gat atg aac cgt gtc tcg 1392
Phe Thr Asn Glu Arg Met His Lys Tyr Arg Asp Met Asn Arg Val Ser
    450                 455                 460

tcc aac att gtg agg ctt gct gat gat atg gga aca tca ttg gct gag 1440
Ser Asn Ile Val Arg Leu Ala Asp Asp Met Gly Thr Ser Leu Ala Glu
465                 470                 475                 480

gtg gag aga ggg gac gtg ccg aaa gca att caa tgc tac atg aat gag 1488
Val Glu Arg Gly Asp Val Pro Lys Ala Ile Gln Cys Tyr Met Asn Glu
                485                 490                 495

acg aat gct tct gaa gaa gaa gca aga gaa tat gta aga aga gtc ata 1536
Thr Asn Ala Ser Glu Glu Glu Ala Arg Glu Tyr Val Arg Arg Val Ile
            500                 505                 510

cag gaa gaa tgg gaa aag ttg aac aca gaa ttg atg cgg gat gat gat 1584
Gln Glu Glu Trp Glu Lys Leu Asn Thr Glu Leu Met Arg Asp Asp Asp
        515                 520                 525

gat gat gat gat ttt aca cta tcc aaa tat tac tgt gag gtg gtt gct 1632
Asp Asp Asp Asp Phe Thr Leu Ser Lys Tyr Tyr Cys Glu Val Val Ala
    530                 535                 540

aat ctt aca aga atg gca cag ttt ata tac caa gat gga tcg gat ggc 1680
Asn Leu Thr Arg Met Ala Gln Phe Ile Tyr Gln Asp Gly Ser Asp Gly
545                 550                 555                 560

ttc ggc atg aaa gat tcc aag gtt aat aga ctg cta aaa gag acg ttg 1728
Phe Gly Met Lys Asp Ser Lys Val Asn Arg Leu Leu Lys Glu Thr Leu
                565                 570                 575

atc gag cgc tac gaa taa 1746
Ile Glu Arg Tyr Glu
            580

<210> 4
<211> 581

<212>  PRT
<213>  Lavandula angustifolia

<400>  4
   Met Ser Thr Ile Ile Ala Ile Gln Val Leu Leu Pro Ile Pro Thr Thr
   1               5                   10                  15
   Lys Thr Tyr Pro Ser His Asp Leu Glu Lys Ser Ser Ser Arg Cys Arg
               20                  25                  30
   Ser Ser Ser Thr Pro Arg Pro Arg Leu Cys Cys Ser Leu Gln Val Ser
           35                  40                  45
   Asp Pro Ile Pro Thr Gly Arg Arg Ser Gly Gly Tyr Pro Pro Ala Leu
       50                  55                  60
   Trp Asp Phe Asp Thr Ile Gln Ser Leu Asn Thr Glu Tyr Lys Gly Glu
   65                  70                  75                  80
   Arg His Met Arg Arg Glu Glu Asp Leu Ile Gly Gln Val Arg Glu Met
                   85                  90                  95
   Leu Val His Glu Val Glu Asp Pro Thr Pro Gln Leu Glu Phe Ile Asp
               100                 105                 110
   Asp Leu His Lys Leu Gly Ile Ser Cys His Phe Glu Asn Glu Ile Leu
           115                 120                 125
   Gln Ile Leu Lys Ser Ile Tyr Leu Asn Gln Asn Tyr Lys Arg Asp Leu
       130                 135                 140
   Tyr Ser Thr Ser Leu Ala Phe Arg Leu Leu Arg Gln Tyr Gly Phe Ile
   145                 150                 155                 160
   Leu Pro Gln Glu Val Phe Asp Cys Phe Lys Asn Glu Glu Gly Thr Asp
                   165                 170                 175
   Phe Lys Pro Ser Phe Gly Arg Asp Ile Lys Gly Leu Leu Gln Leu Tyr
               180                 185                 190
   Glu Ala Ser Phe Leu Ser Arg Lys Gly Glu Glu Thr Leu Gln Leu Ala
           195                 200                 205
   Arg Glu Phe Ala Thr Lys Ile Leu Gln Lys Glu Val Asp Glu Arg Glu
       210                 215                 220
   Phe Ala Thr Lys Met Glu Phe Pro Ser His Trp Thr Val Gln Met Pro
   225                 230                 235                 240
   Asn Ala Arg Pro Phe Ile Asp Ala Tyr Arg Arg Arg Pro Asp Met Asn
                   245                 250                 255
   Pro Val Val Leu Glu Leu Ala Ile Leu Asp Thr Asn Ile Val Gln Ala
               260                 265                 270
   Gln Phe Gln Glu Glu Leu Lys Glu Thr Ser Arg Trp Trp Glu Ser Thr
           275                 280                 285
   Gly Ile Val Gln Glu Leu Pro Phe Val Arg Asp Arg Ile Val Glu Gly
       290                 295                 300
   Tyr Phe Trp Thr Ile Gly Val Thr Gln Arg Arg Glu His Gly Tyr Glu
   305                 310                 315                 320
   Arg Ile Met Thr Ala Lys Val Ile Ala Leu Val Thr Cys Leu Asp Asp
                   325                 330                 335
   Ile Tyr Asp Val Tyr Gly Thr Ile Glu Glu Leu Gln Leu Phe Thr Ser
               340                 345                 350
   Thr Ile Gln Arg Trp Asp Leu Glu Ser Met Lys Gln Leu Pro Thr Tyr
           355                 360                 365
   Met Gln Val Ser Phe Leu Ala Leu His Asn Phe Val Thr Glu Val Ala
       370                 375                 380
   Tyr Asp Thr Leu Lys Lys Lys Gly Tyr Asn Ser Thr Pro Tyr Leu Arg
   385                 390                 395                 400
   Lys Thr Trp Val Asp Leu Val Glu Ser Tyr Ile Lys Glu Ala Thr Trp
                   405                 410                 415
   Tyr Tyr Asn Gly Tyr Lys Pro Ser Met Gln Glu Tyr Leu Asn Asn Ala
               420                 425                 430
   Trp Ile Ser Val Gly Ser Met Ala Ile Leu Asn His Leu Phe Phe Arg
           435                 440                 445
   Phe Thr Asn Glu Arg Met His Lys Tyr Arg Asp Met Asn Arg Val Ser
       450                 455                 460
   Ser Asn Ile Val Arg Leu Ala Asp Asp Met Gly Thr Ser Leu Ala Glu
   465                 470                 475                 480
   Val Glu Arg Gly Asp Val Pro Lys Ala Ile Gln Cys Tyr Met Asn Glu
   485                 490                 495
   Thr Asn Ala Ser Glu Glu Glu Ala Arg Glu Tyr Val Arg Arg Val Ile
               500                 505                 510
   Gln Glu Glu Trp Glu Lys Leu Asn Thr Glu Leu Met Arg Asp Asp Asp
           515                 520                 525
   Asp Asp Asp Asp Phe Thr Leu Ser Lys Tyr Tyr Cys Glu Val Val Ala
       530                 535                 540
   Asn Leu Thr Arg Met Ala Gln Phe Ile Tyr Gln Asp Gly Ser Asp Gly
   545                 550                 555                 560
   Phe Gly Met Lys Asp Ser Lys Val Asn Arg Leu Leu Lys Glu Thr Leu
                   565                 570                 575
   Ile Glu Arg Tyr Glu
               580

<210>  5
<211>  1194
<212>  DNA
<213>  Fusarium poae

<220>
<221>  CDS
<222>  (1)..(1194)
<223>

<400>  5
atg gag aac ttt ccc acc gag tat ttt ctc aac act tct gtg cgc ctt      48
Met Glu Asn Phe Pro Thr Glu Tyr Phe Leu Asn Thr Ser Val Arg Leu
1               5                   10                  15

ctt gag tac att cga tac cga gat agt aac tac acc cga gaa gag cgc      96
Leu Glu Tyr Ile Arg Tyr Arg Asp Ser Asn Tyr Thr Arg Glu Glu Arg
            20                  25                  30

atc gag aat ctg cac tat gct tac aac aag gct gct cat cac ttt gct      144
Ile Glu Asn Leu His Tyr Ala Tyr Asn Lys Ala Ala His His Phe Ala
        35                  40                  45

cag ccc cga caa cag cag cta ctc agg gta gac cct aag cga cta cag      192
Gln Pro Arg Gln Gln Gln Leu Leu Arg Val Asp Pro Lys Arg Leu Gln
    50                  55                  60

gct tcc ctc caa aca atc gtt ggc atg gtc gta tac agt tgg gca aag      240
Ala Ser Leu Gln Thr Ile Val Gly Met Val Val Tyr Ser Trp Ala Lys
65                  70                  75                  80

gtc tct aaa gag tgt atg gcg gct cta tct att cat tac acg tac acg      288
Val Ser Lys Glu Cys Met Ala Ala Leu Ser Ile His Tyr Thr Tyr Thr
                85                  90                  95

ctc gtc ttg gat gac agc agc gac gac cca tat cca gcc atg atg aac      336

Leu Val Leu Asp Asp Ser Ser Asp Asp Pro Tyr Pro Ala Met Met Asn
            100                 105                 110

tat ttc aac gat ctt cag gcc gga cga gaa cag gct cac ccg tgg tgg 384
Tyr Phe Asn Asp Leu Gln Ala Gly Arg Glu Gln Ala His Pro Trp Trp
        115                 120                 125

gcg ctt gtc aat gag cac ttt ccc aat gtc ctt cga cat ttt ggt ccc 432
Ala Leu Val Asn Glu His Phe Pro Asn Val Leu Arg His Phe Gly Pro
    130                 135                 140

ttc tgc tcg ttg aac ctt atc cgc agc act ctc gac tgt aag tac act 480
Phe Cys Ser Leu Asn Leu Ile Arg Ser Thr Leu Asp Cys Lys Tyr Thr
145                 150                 155                 160

gga tac att gtt tca cca cga cat gaa act gac agt tat gga att aaa 528
Gly Tyr Ile Val Ser Pro Arg His Glu Thr Asp Ser Tyr Gly Ile Lys
                165                 170                 175

gtt ttt gag gga tgc tgg atc gag cag tac agc ttt gga ggt ttc cca 576
Val Phe Glu Gly Cys Trp Ile Glu Gln Tyr Ser Phe Gly Gly Phe Pro
            180                 185                 190

gga tcc cat gac tat cct cag ttt ctt cga cgc atg aat ggc ttg ggt 624
Gly Ser His Asp Tyr Pro Gln Phe Leu Arg Arg Met Asn Gly Leu Gly
        195                 200                 205

cac tgt gtc ggg gct tct ttg tgg ccc aag gag cag ttt gat gaa cga 672
His Cys Val Gly Ala Ser Leu Trp Pro Lys Glu Gln Phe Asp Glu Arg
    210                 215                 220

agt cta ttc ctt gaa atc aca tca gcc att gct cag atg gag aac tgg 720
Ser Leu Phe Leu Glu Ile Thr Ser Ala Ile Ala Gln Met Glu Asn Trp
225                 230                 235                 240

atg gtc tgg gtc aac gat ctt atg tca ttc tac aag gaa ttc gac gat 768
Met Val Trp Val Asn Asp Leu Met Ser Phe Tyr Lys Glu Phe Asp Asp
                245                 250                 255

gag cgt gac cag atc agc ctc gtc aag aac tac gtc gtc tct gat gaa 816
Glu Arg Asp Gln Ile Ser Leu Val Lys Asn Tyr Val Val Ser Asp Glu
            260                 265                 270

atc agt ctc cac gaa gct ttg gag aag ctg acc caa gac act ctg cac 864
Ile Ser Leu His Glu Ala Leu Glu Lys Leu Thr Gln Asp Thr Leu His
        275                 280                 285

tcg tcc aag cag atg gtt gct gtc ttc tct gac aag gac cct cag gtg 912
Ser Ser Lys Gln Met Val Ala Val Phe Ser Asp Lys Asp Pro Gln Val
    290                 295                 300

atg gac acg att gag tgc ttc atg cat ggt tat gtc acg tgg cac ttg 960
Met Asp Thr Ile Glu Cys Phe Met His Gly Tyr Val Thr Trp His Leu
305                 310                 315                 320

tgc gat cac agg tac cgc ctt aat gag atc tac gaa aag gtc aag gaa 1008
Cys Asp His Arg Tyr Arg Leu Asn Glu Ile Tyr Glu Lys Val Lys Glu
                325                 330                 335

caa aag acc gag gac gct cag aag ttt tgc aag ttt tat gag cag gct 1056
Gln Lys Thr Glu Asp Ala Gln Lys Phe Cys Lys Phe Tyr Glu Gln Ala
            340                 345                 350

gct aat gtc ggt gcc gtc tcg ccc tcg gag tgg gct tac cca cca gtt      1104
Ala Asn Val Gly Ala Val Ser Pro Ser Glu Trp Ala Tyr Pro Pro Val
        355                 360                 365

gcg caa ctg gca aac gtt cga tcc aag gat gtg aag aat gtg aag cag      1152
Ala Gln Leu Ala Asn Val Arg Ser Lys Asp Val Lys Asn Val Lys Gln
    370                 375                 380

att gaa aag cct ctg ctg agt tca att gag cta gtg gag tga              1194
Ile Glu Lys Pro Leu Leu Ser Ser Ile Glu Leu Val Glu
385                 390                 395

<210>  6
<211>  397
<212>  PRT
<213>  Fusarium poae

<400>  6

Met Glu Asn Phe Pro Thr Glu Tyr Phe Leu Asn Thr Ser Val Arg Leu
1               5                   10                  15

Leu Glu Tyr Ile Arg Tyr Arg Asp Ser Asn Tyr Thr Arg Glu Glu Arg
            20                  25                  30

Ile Glu Asn Leu His Tyr Ala Tyr Asn Lys Ala Ala His His Phe Ala
        35                  40                  45

Gln Pro Arg Gln Gln Gln Leu Leu Arg Val Asp Pro Lys Arg Leu Gln
    50                  55                  60

Ala Ser Leu Gln Thr Ile Val Gly Met Val Val Tyr Ser Trp Ala Lys
65                  70                  75                  80

Val Ser Lys Glu Cys Met Ala Ala Leu Ser Ile His Tyr Thr Tyr Thr
                85                  90                  95

Leu Val Leu Asp Asp Ser Ser Asp Asp Pro Tyr Pro Ala Met Met Asn
            100                 105                 110

Tyr Phe Asn Asp Leu Gln Ala Gly Arg Glu Gln Ala His Pro Trp Trp
        115                 120                 125

Ala Leu Val Asn Glu His Phe Pro Asn Val Leu Arg His Phe Gly Pro
    130                 135                 140

Phe Cys Ser Leu Asn Leu Ile Arg Ser Thr Leu Asp Cys Lys Tyr Thr
145                 150                 155                 160

Gly Tyr Ile Val Ser Pro Arg His Glu Thr Asp Ser Tyr Gly Ile Lys
                165                 170                 175

Val Phe Glu Gly Cys Trp Ile Glu Gln Tyr Ser Phe Gly Gly Phe Pro
            180                 185                 190

Gly Ser His Asp Tyr Pro Gln Phe Leu Arg Arg Met Asn Gly Leu Gly
        195                 200                 205

His Cys Val Gly Ala Ser Leu Trp Pro Lys Glu Gln Phe Asp Glu Arg
    210                 215                 220

Ser Leu Phe Leu Glu Ile Thr Ser Ala Ile Ala Gln Met Glu Asn Trp
225                 230                 235                 240

Met Val Trp Val Asn Asp Leu Met Ser Phe Tyr Lys Glu Phe Asp Asp
                245                 250                 255

Glu Arg Asp Gln Ile Ser Leu Val Lys Asn Tyr Val Val Ser Asp Glu
            260                 265                 270

Ile Ser Leu His Glu Ala Leu Glu Lys Leu Thr Gln Asp Thr Leu His
        275                 280                 285

Ser Ser Lys Gln Met Val Ala Val Phe Ser Asp Lys Asp Pro Gln Val
    290                 295                 300

Met Asp Thr Ile Glu Cys Phe Met His Gly Tyr Val Thr Trp His Leu
305                 310                 315                 320

Cys Asp His Arg Tyr Arg Leu Asn Glu Ile Tyr Glu Lys Val Lys Glu
                325                 330                 335

Gln Lys Thr Glu Asp Ala Gln Lys Phe Cys Lys Phe Tyr Glu Gln Ala
            340                 345                 350

Ala Asn Val Gly Ala Val Ser Pro Ser Glu Trp Ala Tyr Pro Pro Val
        355                 360                 365

Ala Gln Leu Ala Asn Val Arg Ser Lys Asp Val Lys Asn Val Lys Gln
    370                 375                 380

Ile Glu Lys Pro Leu Leu Ser Ser Ile Glu Leu Val Glu
385                 390                 395

<210>  7
<211>  480
<212>  DNA
<213>  Thermococcus sp.

<220>

<221>   CDS
<222>   (1)..(480)
<223>

<400>   7
atg aaa gtt gaa gct ggt gat tat gtt ctc ttc cac tac gtt gga agg        48
Met Lys Val Glu Ala Gly Asp Tyr Val Leu Phe His Tyr Val Gly Arg
1               5                   10                  15

ttc gag gat gga gaa gtt ttt gac aca agc tac gag gag ata gcc aga        96
Phe Glu Asp Gly Glu Val Phe Asp Thr Ser Tyr Glu Glu Ile Ala Arg
            20                  25                  30

gag aat ggc att ctc gtc gag gag agg gag tac ggc cca atg tgg gtc       144
Glu Asn Gly Ile Leu Val Glu Glu Arg Glu Tyr Gly Pro Met Trp Val
        35                  40                  45

agg ata ggc gtc ggt gag atc atc cct ggc ctc gat gaa gcc ata att       192
Arg Ile Gly Val Gly Glu Ile Ile Pro Gly Leu Asp Glu Ala Ile Ile
    50                  55                  60

ggc atg gaa gct gga gag aag aag acc gtg acc gtt ccc ccc gag aag       240
Gly Met Glu Ala Gly Glu Lys Lys Thr Val Thr Val Pro Pro Glu Lys
65                  70                  75                  80

gct tac gga atg ccg aac cca gag ctt gta atc tcc gtt cca agg gaa       288
Ala Tyr Gly Met Pro Asn Pro Glu Leu Val Ile Ser Val Pro Arg Glu
                85                  90                  95

gaa ttc aca aag gcc ggc ctt gaa ccc cag gaa ggt ctc tac gtc atg       336
Glu Phe Thr Lys Ala Gly Leu Glu Pro Gln Glu Gly Leu Tyr Val Met
            100                 105                 110

acc gat tct ggc ata gcc aag ata gtt tcc gtt gga gag agc gag gta       384
Thr Asp Ser Gly Ile Ala Lys Ile Val Ser Val Gly Glu Ser Glu Val
        115                 120                 125

tcc ctt gac ttc aac cac ccg cta gca ggt aag acc cta gtc ttt gag       432
Ser Leu Asp Phe Asn His Pro Leu Ala Gly Lys Thr Leu Val Phe Glu
    130                 135                 140

gta gaa gtc ata gaa gta aaa aag gcc gaa gag gac tca gaa gct tag       480
Val Glu Val Ile Glu Val Lys Lys Ala Glu Glu Asp Ser Glu Ala
145                 150                 155


<210>   8
<211>   159
<212>   PRT
<213>   Thermococcus sp.

<400>   8

Met Lys Val Glu Ala Gly Asp Tyr Val Leu Phe His Tyr Val Gly Arg
1               5                   10                  15


Phe Glu Asp Gly Glu Val Phe Asp Thr Ser Tyr Glu Glu Ile Ala Arg
            20                  25                  30


Glu Asn Gly Ile Leu Val Glu Glu Arg Glu Tyr Gly Pro Met Trp Val
        35                  40                  45

Arg Ile Gly Val Gly Glu Ile Ile Pro Gly Leu Asp Glu Ala Ile Ile
    50                  55                  60

Gly Met Glu Ala Gly Glu Lys Lys Thr Val Thr Val Pro Pro Glu Lys
65                  70                  75                  80

Ala Tyr Gly Met Pro Asn Pro Glu Leu Val Ile Ser Val Pro Arg Glu
                85                  90                  95

Glu Phe Thr Lys Ala Gly Leu Glu Pro Gln Glu Gly Leu Tyr Val Met
            100                 105                 110

Thr Asp Ser Gly Ile Ala Lys Ile Val Ser Val Gly Glu Ser Glu Val
        115                 120                 125

Ser Leu Asp Phe Asn His Pro Leu Ala Gly Lys Thr Leu Val Phe Glu
    130                 135                 140

Val Glu Val Ile Glu Val Lys Lys Ala Glu Glu Asp Ser Glu Ala
145                 150                 155

<210>  9
<211>  3369
<212>  DNA
<213>  Artificial

<220>
<223>  Synthetic gene

<400>  9
catatgaaag ttgaagctgg tgattatgtt ctcttccact acgttggaag gttcgaggat      60

ggagaagttt ttgacacaag ctacgaggag atagccagag agaatggcat tctcgtcgag     120

gagagggagt acggcccaat gtgggtcagg ataggcgtcg gtgagatcat ccctggcctc     180

gatgaagcca taattggcat ggaagctgga gagaagaaga ccgtgaccgt tccccccgag     240

aaggcttacg gaatgccgaa cccagagctt gtaatctccg ttccaaggga agaattcaca     300

aaggccggcc ttgaacccca ggaaggtctc tacgtcatga ccgattctgg catagccaag     360

atagtttccg ttggagagag cgaggtatcc cttgacttca accacccgct agcaggtaag     420

accctagtct ttgaggtaga agtcatagaa gtaaaaaagg ccgaagagga ctcagaagct     480

ggaggtggag gttctggagg tggaggttct ggaggtggag gttctggagg tggaggttct     540

ggaggtggat ctgaaaccag aaggtctgcc aattatgaac caaatagctg ggattatgat     600

tatttgctgt cttctgacac tgacgaatct attgaagtat acaaagacaa ggccaaaaag     660

ctggaggctg aggtgagaag agagattaac aatgaaaagg cagagttttt gactctgcct     720

gaactgatag ataatgttca aaggttagga ttaggttaca gattcgagag tgacataagg 780
agagcccttg atagatttgt ttcttcagga ggatttgatg ctgttacaaa aactagcctt 840
catgctactg ctcttagctt caggcttctc agacagcatg gctttgaggt atctcaagaa 900
gctttcagcg gattcaagga tcaaaatggc aatttcttga aaaaccttaa ggaggacatc 960
aaggcaatac taagcctata tgaagcttca tttcttgcct tagaaggaga aaatatcttg 1020
gatgaggcca aggtgtttgc aatatcacat ctaaaagagc ttagcgaaga aaagattgga 1080
aaagacctgg ccgaacaggt gaatcatgca ttggagcttc cattgcatag aaggacacaa 1140
agactagaag ctgtttggag cattgaagca tacagaaaaa aggaagatgc agatcaagta 1200
ctgctagaac ttgctatatt ggactacaac atgattcaat cagtatacca aagagatctt 1260
agagagacat caaggtggtg gaggagagtg ggtcttgcaa caaagttgca ttttgctaga 1320
gacaggttaa ttgaaagctt ttactgggca gttggagttg catttgaacc tcaatacagt 1380
gattgtagaa attccgtagc aaaaatgttt tcttttgtaa caatcattga tgatatctat 1440
gatgtttatg gtactctgga tgagttggag ctatttacag atgctgttga gagatgggat 1500
gttaatgcca tcgatgatct tcctgattat atgaagcttt gtttcctagc tctttataac 1560
actatcaatg agatagctta tgataatctg aaggacaagg gggaaaacat tcttccatac 1620
ctaacaaaag catgggcaga tttatgtaat gcattcctac aagaagcaaa atggttgtac 1680
aataagtcca caccaacatt tgatgaatat ttcggaaatg catggaaatc atcctcaggg 1740
cctcttcaac tagtttttgc ctactttgcc gttgttcaaa acatcaagaa agaggaaatt 1800
gataacttac aaaagtatca tgatatcatc agtaggcctt cccatatctt tagactttgt 1860
aacgacttgg cttcagcatc tgctgagata gcaagaggtg aaaccgcaaa ttctgtatca 1920
tgttacatga gaacaaaagg catttctgag gaacttgcta ctgaatccgt aatgaatttg 1980
atcgacgaaa cctggaaaaa gatgaacaaa gaaaagcttg gtggctctct gtttgcaaaa 2040
ccttttgttg aaacagctat taaccttgca agacaatccc attgtactta tcataacgga 2100
gatgcacata cttcaccaga tgagcttact aggaaaagag tactgtcagt aatcacagag 2160
cctattctac cttttgagag aggcggtagc caccatcacc atcaccatta ataattttaa 2220
aatataagtg atttagatat tcataatata tttgggaggt aaattaatat gaccagcgcc 2280
caacgtaagg acgaccatgt aaggctcgcc atcgagcagc ataacgccca tagcggacgt 2340
aaccagttcg acgacgtgtc tttcgtacat catgccctgg ccggcatcga caggccagac 2400
gtgtccctgg ccacgtcctt cgccgggatc tcctggcagg tgcctatcta catcaacgcg 2460
atgaccggcg gcagcgagaa gaccggcctc atcaacaggg acctggccac cgccgcccgt 2520
gagaccggcg tacccatcgc gtccgggtcc atgaacgcgt acatcaagga cccctcctgc 2580
gccgacacgt tccgtgtgct gcgtgacgag aaccccaacg ggttcgtaat cgcgaacatc 2640

aacgccacca cgacggttga caacgcgcag cgtgcgatcg acctgatcga ggcgaacgcc 2700

ctgcagatcc atatcaacac ggcgcaggag acgcctatgc ctgagggcga caggtctttc 2760

gcgtcctggg tccctcagat cgagaagatc gcggcggccg tagacatccc cgtgatcgta 2820

aaggaggtag gcaacggcct gagcaggcag accatcctgc tgctcgccga cctcggcgtg 2880

caggcggcgg acgtaagcgg ccgtggcggc acggacttcg cccgtatcga gaacggccgt 2940

agggagctcg gcgactacgc gttcctgcat ggctgggggc agtccaccgc cgcctgcctg 3000

ctggacgccc aggacatctc cctgcccgta ctcgcctccg gcggtgtgcg tcatcctctc 3060

gacgtggtac gtgccctcgc gctcggcgcc cgtgccgtag gctcctccgc cggcttcctg 3120

cgtaccctga tggacgacgg cgtagacgcg ctgatcacga agctcacgac ctggctggac 3180

cagctggcgg cgctgcagac catgctcggc gcgcgtaccc ctgccgacct cacccgttgc 3240

gacgtgctgc tccatggcga gctgcgtgac ttctgcgccg acaggggcat cgacacgcgt 3300

cgtctcgccc agcgttccag ctccatcgag gccctccaga cgacgggaag cacaagataa 3360

taaggatcc 3369

<210> 10
<211> 2796
<212> DNA
<213> Artificial

<220>
<223> Synthetic gene

<400> 10
catatggaaa ccagaaggtc tgccaattat gaaccaaata gctgggatta tgattatttg 60

ctgtcttctg acactgacga atctattgaa gtatacaaag acaaggccaa aaagctggag 120

gctgaggtga gaagagagat taacaatgaa aaggcagagt ttttgactct gcctgaactg 180

atagataatg ttcaaaggtt aggattaggt tacagattcg agagtgacat aaggagagcc 240

cttgatagat ttgtttcttc aggaggattt gatgctgtta caaaaactag ccttcatgct 300

actgctctta gcttcaggct tctcagacag catggctttg aggtatctca agaagctttc 360

agcggattca aggatcaaaa tggcaatttc ttgaaaaacc ttaaggagga catcaaggca 420

atactaagcc tatatgaagc ttcatttctt gccttagaag gagaaaatat cttggatgag 480

gccaaggtgt ttgcaatatc acatctaaaa gagcttagcg aagaaaagat tggaaaagac 540

ctggccgaac aggtgaatca tgcattggag cttccattgc atagaaggac acaaagacta 600

gaagctgttt ggagcattga agcatacaga aaaaaggaag atgcagatca agtactgcta 660

gaacttgcta tattggacta caacatgatt caatcagtat accaaagaga tcttagagag 720

acatcaaggt ggtggaggag agtgggtctt gcaacaaagt tgcattttgc tagagacagg 780

ttaattgaaa gcttttactg ggcagttgga gttgcatttg aacctcaata cagtgattgt 840
agaaattccg tagcaaaaat gttttctttt gtaacaatca ttgatgatat ctatgatgtt 900
tatggtactc tggatgagtt ggagctattt acagatgctg ttgagagatg ggatgttaat 960
gccatcgatg atcttcctga ttatatgaag ctttgtttcc tagctcttta taacactatc 1020
aatgagatag cttatgataa tctgaaggac aagggggaaa acattcttcc atacctaaca 1080
aaagcatggg cagatttatg taatgcattc ctacaagaag caaaatggtt gtacaataag 1140
tccacaccaa catttgatga atatttcgga aatgcatgga aatcatcctc agggcctctt 1200
caactagttt ttgcctactt tgccgttgtt caaaacatca agaaagagga aattgataac 1260
ttacaaaagt atcatgatat catcagtagg ccttcccata tctttagact ttgtaacgac 1320
ttggcttcag catctgctga gatagcaaga ggtgaaaccg caaattctgt atcatgttac 1380
atgagaacaa aaggcatttc tgaggaactt gctactgaat ccgtaatgaa tttgatcgac 1440
gaaacctgga aaaagatgaa caaagaaaag cttggtggct ctctgtttgc aaaacctttt 1500
gttgaaacag ctattaacct tgcaagacaa tcccattgta cttatcataa cggagatgca 1560
catacttcac cagatgagct tactaggaaa agagtactgt cagtaatcac agagcctatt 1620
ctaccttttg agagataata attttaaaat ataagtgatt tagatattca taatatattt 1680
gggaggtaaa ttaatatgac cagcgcccaa cgtaaggacg accatgtaag gctcgccatc 1740
gagcagcata acgcccatag cggacgtaac cagttcgacg acgtgtcttt cgtacatcat 1800
gccctggccg gcatcgacag gccagacgtg tccctggcca cgtccttcgc cgggatctcc 1860
tggcaggtgc ctatctacat caacgcgatg accggcggca gcgagaagac cggcctcatc 1920
aacagggacc tggccaccgc cgcccgtgag accggcgtac ccatcgcgtc cgggtccatg 1980
aacgcgtaca tcaaggaccc ctcctgcgcc gacacgttcc gtgtgctgcg tgacgagaac 2040
cccaacgggt tcgtaatcgc gaacatcaac gccaccacga cggttgacaa cgcgcagcgt 2100
gcgatcgacc tgatcgaggc gaacgccctg cagatccata tcaacacggc gcaggagacg 2160
cctatgcctg agggcgacag gtctttcgcg tcctgggtcc ctcagatcga gaagatcgcg 2220
gcggccgtag acatccccgt gatcgtaaag gaggtaggca acggcctgag caggcagacc 2280
atcctgctgc tcgccgacct cggcgtgcag gcggcggacg taagcggccg tggcggcacg 2340
gacttcgccc gtatcgagaa cggccgtagg gagctcggcg actacgcgtt cctgcatggc 2400
tgggggcagt ccaccgccgc ctgcctgctg gacgcccagg acatctccct gcccgtactc 2460
gcctccggcg gtgtgcgtca tcctctcgac gtggtacgtg ccctcgcgct cggcgcccgt 2520
gccgtaggct cctccgccgg cttcctgcgt accctgatgg acgacggcgt agacgcgctg 2580
atcacgaagc tcacgacctg gctggaccag ctggcggcgc tgcagaccat gctcggcgcg 2640
cgtacccctg ccgacctcac ccgttgcgac gtgctgctcc atggcgagct gcgtgacttc 2700

tgcgccgaca ggggcatcga cacgcgtcgt ctcgcccagc gttccagctc catcgaggcc 2760

ctccagacga cgggaagcac aagataataa ggatcc 2796

<210> 11
<211> 3726
<212> DNA
<213> Artificial

<220>
<223> Synthetic gene

<400> 11
cccgggttca gattaaattt ttgcttattt gatttacatt atataatatt gagtaaagta 60

ttgactagca aaattttttg atactttaat ttgtgaaatt tcttatcaaa agttatattt 120

ttgaatgatt tttattgaaa aatacaacta aaaaggatta tagtataagt gtgtgtaatt 180

ttgtgttaaa tttaaaggga ggaaatgaac atgaaagttg aagctggtga ttatgttctc 240

ttccactacg ttggaaggtt cgaggatgga gaagtttttg acacaagcta cgaggagata 300

gccagagaga atggcattct cgtcgaggag agggagtacg gcccaatgtg ggtcaggata 360

ggcgtcggtg agatcatccc tggcctcgat gaagccataa ttggcatgga agctggagag 420

aagaagaccg tgaccgttcc ccccgagaag gcttacggaa tgccgaaccc agagcttgta 480

atctccgttc caagggaaga attcacaaag gccggccttg aaccccagga aggtctctac 540

gtcatgaccg attctggcat agccaagata gtttccgttg gagagagcga ggtatccctt 600

gacttcaacc acccgctagc aggtaagacc ctagtctttg aggtagaagt catagaagta 660

aaaaaggccg aagaggactc agaagctgga ggtggaggtt ctggaggtgg aggttctgga 720

ggtggaggtt ctggaggtgg aggttctgga ggtggatctg aaaccagaag gtctgccaat 780

tatgaaccaa atagctggga ttatgattat ttgctgtctt ctgacactga cgaatctatt 840

gaagtataca aagacaaggc caaaaagctg gaggctgagg tgagaagaga gattaacaat 900

gaaaaggcag agtttttgac tctgcctgaa ctgatagata atgttcaaag gttaggatta 960

ggttacagat tcgagagtga cataaggaga gcccttgata gatttgtttc ttcaggagga 1020

tttgatgctg ttacaaaaac tagccttcat gctactgctc ttagcttcag gcttctcaga 1080

cagcatggct ttgaggtatc tcaagaagct ttcagcggat tcaaggatca aaatggcaat 1140

ttcttgaaaa accttaagga ggacatcaag gcaatactaa gcctatatga agcttcattt 1200

cttgccttag aaggagaaaa tatcttggat gaggccaagg tgtttgcaat atcacatcta 1260

aaagagctta gcgaagaaaa gattggaaaa gacctggccg aacaggtgaa tcatgcattg 1320

gagcttccat tgcatagaag gacacaaaga ctagaagctg tttggagcat tgaagcatac 1380

agaaaaaagg aagatgcaga tcaagtactg ctagaacttg ctatattgga ctacaacatg 1440

attcaatcag tataccaaag agatcttaga gagacatcaa ggtggtggag gagagtgggt 1500
cttgcaacaa agttgcattt tgctagagac aggttaattg aaagctttta ctgggcagtt 1560
ggagttgcat ttgaacctca atacagtgat tgtagaaatt ccgtagcaaa aatgttttct 1620
tttgtaacaa tcattgatga tatctatgat gtttatggta ctctggatga gttggagcta 1680
tttacagatg ctgttgagag atgggatgtt aatgccatcg atgatcttcc tgattatatg 1740
aagctttgtt tcctagctct ttataacact atcaatgaga tagcttatga taatctgaag 1800
gacaaggggg aaaacattct tccataccta acaaaagcat gggcagattt atgtaatgca 1860
ttcctacaag aagcaaaatg gttgtacaat aagtccacac caacatttga tgaatatttc 1920
ggaaatgcat ggaaatcatc ctcagggcct cttcaactag tttttgccta ctttgccgtt 1980
gttcaaaaca tcaagaaaga ggaaattgat aacttacaaa agtatcatga tatcatcagt 2040
aggccttccc atatctttag actttgtaac gacttggctt cagcatctgc tgagatagca 2100
agaggtgaaa ccgcaaattc tgtatcatgt tacatgagaa caaaaggcat ttctgaggaa 2160
cttgctactg aatccgtaat gaatttgatc gacgaaacct ggaaaaagat gaacaaagaa 2220
aagcttggtg gctctctgtt tgcaaaacct tttgttgaaa cagctattaa ccttgcaaga 2280
caatcccatt gtacttatca taacggagat gcacatactt caccagatga gcttactagg 2340
aaaagagtac tgtcagtaat cacagagcct attctacctt ttgagagagg cggtagccac 2400
catcaccatc accattaata attttaaaat ataagtgatt tagatattca taatatattt 2460
gggaggtaaa ttaatatgac cagcgcccaa cgtaaggacg accatgtaag gctcgccatc 2520
gagcagcata acgcccatag cggacgtaac cagttcgacg acgtgtcttt cgtacatcat 2580
gccctggccg gcatcgacag gccagacgtg tccctggcca cgtccttcgc cgggatctcc 2640
tggcaggtgc ctatctacat caacgcgatg accggcggca gcgagaagac cggcctcatc 2700
aacagggacc tggccaccgc cgcccgtgag accggcgtac ccatcgcgtc cgggtccatg 2760
aacgcgtaca tcaaggaccc ctcctgcgcc gacacgttcc gtgtgctgcg tgacgagaac 2820
cccaacgggt tcgtaatcgc gaacatcaac gccaccacga cggttgacaa cgcgcagcgt 2880
gcgatcgacc tgatcgaggc gaacgccctg cagatccata tcaacacggc gcaggagacg 2940
cctatgcctg agggcgacag gtctttcgcg tcctgggtcc ctcagatcga gaagatcgcg 3000
gcggccgtag acatccccgt gatcgtaaag gaggtaggca acggcctgag caggcagacc 3060
atcctgctgc tcgccgacct cggcgtgcag gcggcggacg taagcggccg tggcggcacg 3120
gacttcgccc gtatcgagaa cggccgtagg gagctcggcg actacgcgtt cctgcatggc 3180
tgggggcagt ccaccgccgc ctgcctgctg gacgcccagg acatctccct gcccgtactc 3240
gcctccggcg gtgtgcgtca tcctctcgac gtggtacgtg ccctcgcgct cggcgcccgt 3300
gccgtaggct cctccgccgg cttcctgcgt accctgatgg acgacggcgt agacgcgctg 3360

atcacgaagc tcacgacctg gctggaccag ctggcggcgc tgcagaccat gctcggcgcg 3420

cgtacccctg ccgacctcac ccgttgcgac gtgctgctcc atggcgagct gcgtgacttc 3480

tgcgccgaca ggggcatcga cacgcgtcgt ctcgcccagc gttccagctc catcgaggcc 3540

ctccagacga cgggaagcac aagataataa tttaaaagca aatataaatg aaaaattgaa 3600

ccctagcatt atgtaaatgc agggtttaat ttttatatta agcagcataa tagaaagttt 3660

tttaaatgca tgtatatatg gggtatttaa agggaaatct ataatataat ttaggactat 3720

tccgga 3726

<210> 12
<211> 3153
<212> DNA
<213> Artificial

<220>
<223> Synthetic gene

<400> 12
cccgggttca gattaaattt ttgcttattt gatttacatt atataatatt gagtaaagta 60

ttgactagca aaattttttg atactttaat ttgtgaaatt tcttatcaaa agttatattt 120

ttgaatgatt tttattgaaa aatacaacta aaaaggatta tagtataagt gtgtgtaatt 180

ttgtgttaaa tttaaaggga ggaaatgaac atggaaacca gaaggtctgc caattatgaa 240

ccaaatagct gggattatga ttatttgctg tcttctgaca ctgacgaatc tattgaagta 300

tacaaagaca aggccaaaaa gctggaggct gaggtgagaa gagagattaa caatgaaaag 360

gcagagtttt tgactctgcc tgaactgata gataatgttc aaaggttagg attaggttac 420

agattcgaga gtgacataag gagagccctt gatagatttg tttcttcagg aggatttgat 480

gctgttacaa aaactagcct tcatgctact gctcttagct tcaggcttct cagacagcat 540

ggctttgagg tatctcaaga agctttcagc ggattcaagg atcaaaatgg caatttcttg 600

aaaaacctta aggaggacat caaggcaata ctaagcctat atgaagcttc atttcttgcc 660

ttagaaggag aaaatatctt ggatgaggcc aaggtgtttg caatatcaca tctaaaagag 720

cttagcgaag aaaagattgg aaaagacctg gccgaacagg tgaatcatgc attggagctt 780

ccattgcata gaaggacaca aagactagaa gctgtttgga gcattgaagc atacagaaaa 840

aaggaagatg cagatcaagt actgctagaa cttgctatat tggactacaa catgattcaa 900

tcagtatacc aaagagatct tagagagaca tcaaggtggt ggaggagagt gggtcttgca 960

acaaagttgc attttgctag agacaggtta attgaaagct tttactgggc agttggagtt 1020

gcatttgaac ctcaatacag tgattgtaga aattccgtag caaaaatgtt ttcttttgta 1080

acaatcattg atgatatcta tgatgtttat ggtactctgg atgagttgga gctatttaca 1140

gatgctgttg agagatggga tgttaatgcc atcgatgatc ttcctgatta tatgaagctt 1200
tgtttcctag ctctttataa cactatcaat gagatagctt atgataatct gaaggacaag 1260
ggggaaaaca ttcttccata cctaacaaaa gcatgggcag atttatgtaa tgcattccta 1320
caagaagcaa aatggttgta caataagtcc acaccaacat ttgatgaata tttcggaaat 1380
gcatggaaat catcctcagg gcctcttcaa ctagtttttg cctactttgc cgttgttcaa 1440
aacatcaaga aagaggaaat tgataactta caaaagtatc atgatatcat cagtaggcct 1500
tcccatatct ttagactttg taacgacttg gcttcagcat ctgctgagat agcaagaggt 1560
gaaaccgcaa attctgtatc atgttacatg agaacaaaag gcatttctga ggaacttgct 1620
actgaatccg taatgaattt gatcgacgaa acctggaaaa agatgaacaa agaaaagctt 1680
ggtggctctc tgtttgcaaa accttttgtt gaaacagcta ttaaccttgc aagacaatcc 1740
cattgtactt atcataacgg agatgcacat acttcaccag atgagcttac taggaaaaga 1800
gtactgtcag taatcacaga gcctattcta ccttttgaga gataataatt ttaaaatata 1860
agtgatttag atattcataa tatatttggg aggtaaatta atatgaccag cgcccaacgt 1920
aaggacgacc atgtaaggct cgccatcgag cagcataacg cccatagcgg acgtaaccag 1980
ttcgacgacg tgtctttcgt acatcatgcc ctggccggca tcgacaggcc agacgtgtcc 2040
ctggccacgt ccttcgccgg gatctcctgg caggtgccta tctacatcaa cgcgatgacc 2100
ggcggcagcg agaagaccgg cctcatcaac agggacctgg ccaccgccgc ccgtgagacc 2160
ggcgtaccca tcgcgtccgg gtccatgaac gcgtacatca aggacccctc ctgcgccgac 2220
acgttccgtg tgctgcgtga cgagaacccc aacgggttcg taatcgcgaa catcaacgcc 2280
accacgacgg ttgacaacgc gcagcgtgcg atcgacctga tcgaggcgaa cgccctgcag 2340
atccatatca acacggcgca ggagacgcct atgcctgagg gcgacaggtc tttcgcgtcc 2400
tgggtccctc agatcgagaa gatcgcggcg gccgtagaca tccccgtgat cgtaaaggag 2460
gtaggcaacg gcctgagcag gcagaccatc ctgctgctcg ccgacctcgg cgtgcaggcg 2520
gcggacgtaa gcggccgtgg cggcacggac ttcgcccgta tcgagaacgg ccgtagggag 2580
ctcggcgact acgcgttcct gcatggctgg gggcagtcca ccgccgcctg cctgctggac 2640
gcccaggaca tctccctgcc cgtactcgcc tccggcggtg tgcgtcatcc tctcgacgtg 2700
gtacgtgccc tcgcgctcgg cgcccgtgcc gtaggctcct ccgccggctt cctgcgtacc 2760
ctgatggacg acggcgtaga cgcgctgatc acgaagctca cgacctggct ggaccagctg 2820
gcggcgctgc agaccatgct cggcgcgcgt acccctgccg acctcacccg ttgcgacgtg 2880
ctgctccatg gcgagctgcg tgacttctgc gccgacaggg gcatcgacac gcgtcgtctc 2940
gcccagcgtt ccagctccat cgaggccctc cagacgacgg gaagcacaag ataataattt 3000
aaaagcaaat ataaatgaaa aattgaaccc tagcattatg taaatgcagg gtttaatttt 3060

tatattaagc agcataatag aaagtttttt aaatgcatgt atatatgggg tatttaaagg 3120

gaaatctata atataattta ggactattcc gga 3153

<210> 13
<211> 3897
<212> DNA
<213> Artificial

<220>
<223> Plasmid vector pSKCL

<400> 13
gacgaaaggg cctcgtgata cgcctatttt tataggttaa tgtcatgata ataatggttt 60

cttagacgtc aggtggcact tttcggggaa atgtgcgcgg aacccctatt tgtttatttt 120

tctaaataca ttcaaatatg tatccgctca tgagacaata accctgataa atgcttcaat 180

aatattgaaa aaggaagagt atgagtattc aacatttccg tgtcgccctt attccctttt 240

ttgcggcatt ttgccttcct gtttttgctc acccagaaac gctggtgaaa gtaaaagatg 300

ctgaagatca gttgggtgca cgagtgggtt acatcgaact ggatctcaac agcggtaaga 360

tccttgagag ttttcgcccc gaagaacgtt ttccaatgat gagcactttt aaagttctgc 420

tatgtggcgc ggtattatcc cgtattgacg ccgggcaaga gcaactcggt cgccgcatac 480

actattctca gaatgacttg gttgagtact caccagtcac agaaaagcat cttacggatg 540

gcatgacagt aagagaatta tgcagtgctg ccataaccat gagtgataac actgcggcca 600

acttacttct gacaacgatc ggaggaccga aggagctaac cgcttttttg cacaacatgg 660

gggatcatgt aactcgcctt gatcgttggg aaccggagct gaatgaagcc ataccaaacg 720

acgagcgtga caccacgatg cctgtagcaa tggcaacaac gttgcgcaaa ctattaactg 780

gcgaactact tactctagct tcccggcaac aattaataga ctggatggag gcggataaag 840

ttgcaggacc acttctgcgc tcggcccttc cggctggctg gtttattgct gataaatctg 900

gagccggtga gcgtgggtct cgcggtatca ttgcagcact ggggccagat ggtaagccct 960

cccgtatcgt agttatctac acgacgggga gtcaggcaac tatggatgaa cgaaatagac 1020

agatcgctga gataggtgcc tcactgatta agcattggta actgtcagac caagtttact 1080

catatatact ttagattgat ttaaaacttc atttttaatt taaaaggatc taggtgaaga 1140

tcctttttga taatctcatg accaaaatcc cttaacgtga gttttcgttc cactgagcgt 1200

cagaccccgt agaaaagatc aaaggatctt cttgagatcc tttttttctg cgcgtaatct 1260

gctgcttgca aacaaaaaaa ccaccgctac cagcggtggt ttgtttgccg gatcaagagc 1320

taccaactct ttttccgaag gtaactggct tcagcagagc gcagatacca aatactgtcc 1380

ttctagtgta gccgtagtta ggccaccact tcaagaactc tgtagcaccg cctacatacc 1440

tcgctctgct aatcctgtta ccagtggctg ctgccagtgg cgataagtcg tgtcttaccg 1500

ggttggactc aagacgatag ttaccggata aggcgcagcg gtcgggctga acggggggtt 1560

cgtgcacaca gcccagcttg gagcgaacga cctacaccga actgagatac ctacagcgtg 1620

agctatgaga aagcgccacg cttcccgaag ggagaaaggc ggacaggtat ccggtaagcg 1680

gcagggtcgg aacaggagag cgcacgaggg agcttccagg gggaaacgcc tggtatcttt 1740

atagtcctgt cgggtttcgc cacctctgac ttgagcgtcg atttttgtga tgctcgtcag 1800

gggggcggag cctatggaaa aacgccagca acgcggcctt tttacggttc ctggcctttt 1860

gctggccttt tgctcacatg ttctttcctg cgttatcccc tgattctgtg gataaccgta 1920

ttaccgcctt tgagtgagct gataccgctc gccgcagccg aacgaccgag cgcagcgagt 1980

cagtgagcga ggaagcggaa gagaattcgg cgcgcccggg ccgcggatcc atatggtacc 2040

tctagacgcg tgcatgctcg agtccggaag cttttcagat taaatttttg cttatttgat 2100

ttacattata taatattgag taaagtattg actagcaaaa ttttttgata ctttaatttg 2160

tgaaatttct tatcaaaagt tatatttttg aatgattttt attgaaaaat acaactaaaa 2220

aggattatag tataagtgtg tgtaattttg tgttaaattt aaagggagga aatgaacatg 2280

aactttaata aaattgattt agacaattgg aagagaaaag agatatttaa tcattatttg 2340

aaccaacaaa cgacttttag tataaccaca gaaattgata ttagtgtttt atacagaaac 2400

ataaaacaag aaggatataa attttaccct gcatttattt tcttagtgac aagggtgata 2460

aactcaaata cagcttttag aactggttac aatagcgacg gagagttagg ttattgggat 2520

aagttagagc cactttatac aatttttgat ggtgtatcta aaacattctc tggtatttgg 2580

actcctgtaa agaatgactt caaagagttt tatgatttat acctttctga tgtagagaaa 2640

tataatggtt cggggaaatt gtttcccaaa acacctatac ctgaaaatgc tttttctctt 2700

tctattattc catggacttc atttactggg tttaacttaa atatcaataa taatagtaat 2760

taccttctac ccattattac agcaggaaaa ttcattaata aaggtaattc aatatattta 2820

ccgctatctt tacaggtaca tcattctgtt tgtgatggtt atcatgcagg attgtttatg 2880

aactctattc aggaattgtc agataggcct aatgactggc ttttataatt taaaagcaaa 2940

tataaatgaa aaattgaacc ctagcattat gtaaatgcag ggtttaattt ttatattaag 3000

cagcataata gaaagttttt taaatgcatg tatatatggg gtatttaaag ggaaatctat 3060

aatataattt aggactattc gaatttttta ttaaaacgtc tcaaaatcgt ttctgagacg 3120

ttttagcgtt tatttcgttt agttatcggc ataatcgtta aaacaggcgt tatcgtagcg 3180

taaaagccct tgagcgtagc gtggctttgc agcgaagatg ttgtctgtta gattatgaaa 3240

gccgatgact gaatgaaata ataagcgcag cgcccttcta tttcggttgg aggaggctca 3300

agggagtatg agggaatgaa attccctcat gggtttgatt ttaaaaattg cttgcaattt 3360

tgccgagcgg tagcgctgga aaatttttga aaaaaatttg gaatttggaa aaaatttggt 3420

ttgatgttgc gattaatagc aatacaattg caataaacaa aatgatcgat gctgtttggc 3480

aaaaaaagaa aaagtgatta atttatattt tatttatggc gctaatttat tacggctttt 3540

tttgttgtcg gctagccgat tctgatacat ttttttaagc acaaaaacca cccaattttg 3600

gagtggtgtg taagtgcgca ttgtcatgaa aaaatggcac gcaatttcat cactttttaa 3660

agtgatgtgt aagtgcgcat tgtcatgaaa aaatggcacg caatttcatc actttttaaa 3720

gtgatgtgta agtgcgcatt gttggaaaaa tcgaactatg atttattttt gctgttgtat 3780

ttatttttca tcttttgggt tttggttttg ttttttgttg ctatcgtagt ttatttgctt 3840

tttaagggct ctatttttcg ttctacggca tttttataat ttgccaatat aatttat 3897

<210> 14
<211> 2509
<212> DNA
<213> Artificial

<220>
<223> Synthetic gene

<400> 14
ggatccttca gattaaattt ttgcttattt gatttacatt atataatatt gagtaaagta 60

ttgactagca aaattttttg atactttaat ttgtgaaatt tcttatcaaa agttatattt 120

ttgaatgatt tttattgaaa aatacaacta aaaaggatta tagtataagt gtgtgtaatt 180

ttgtgttaaa tttaaaggga ggaaatgaac atgaaagttg aagctggtga ttatgttctc 240

ttccactacg ttggaaggtt cgaggatgga gaagtttttg acacaagcta cgaggagata 300

gccagagaga atggcattct cgtcgaggag agggagtacg gcccaatgtg ggtcaggata 360

ggcgtcggtg agatcatccc tggcctcgat gaagccataa ttggcatgga agctggagag 420

aagaagaccg tgaccgttcc ccccgagaag gcttacggaa tgccgaaccc agagcttgta 480

atctccgttc caagggaaga attcacaaag gccggccttg aaccccagga aggtctctac 540

gtcatgaccg attctggcat agccaagata gtttccgttg gagagagcga ggtatccctt 600

gacttcaacc acccgctagc aggtaagacc ctagtctttg aggtagaagt catagaagta 660

aaaaaggccg aagaggactc agaagctgga ggtggaggtt ctggaggtgg aggttctgga 720

ggtggaggtt ctggaggtgg aggttctgga ggtggatcta cgggccggcg atccggaggc 780

tacccgcccg ccctatggga tttcgacact attcaatcgc tcaacaccga gtataaggga 840

gagaggcaca tgagaaggga agaagaccta attgggcaag ttagagagat gctggtgcat 900

gaagtagaag atcccactcc acagctggag ttcattgatg atttgcataa gcttggcata 960

tcttgccatt ttgagaatga aatcctccaa atcttgaaat ccatatatct taatcaaaac 1020

tacaaaaggg atttgtactc aacatctcta gcattcagac tcctcagaca atatggcttc 1080

atccttccac aagaagtatt tgattgtttc aagaatgagg agggtacgga tttcaagcca 1140

agcttcggcc gtgatatcaa aggcttgtta caattgtatg aagcttcttt cctatcaaga 1200

aaaggagaag aaactttaca actagcaaga gagtttgcaa caaagattct gcaaaaagaa 1260

gttgatgaga gagagtttgc aaccaagatg gagttccctt ctcattggac ggttcaaatg 1320

ccgaatgcaa gacctttcat cgatgcttac cgtaggaggc cggatatgaa tccagttgtg 1380

ctcgagctag ccatacttga tacaaatata gttcaagcac aatttcaaga agaactcaaa 1440

gagacctcaa ggtggtggga gagtacaggc attgtccaag agcttccatt tgtgagggat 1500

aggattgtgg aaggctactt ttggacgatt ggagtgactc agagacgcga gcatggatac 1560

gaaagaatca tgaccgcaaa ggttattgcc ttagtaacat gtttagacga catatacgat 1620

gtttatggca cgatagaaga gcttcaactt ttcacaagca caatccaaag atgggatttg 1680

gaatcaatga agcaactccc tacctacatg caagtaagct ttcttgcact acacaacttt 1740

gtaaccgagg tggcttacga tactctcaag aaaaagggct acaactccac accatattta 1800

agaaaaacgt gggtggatct tgttgaatca tatatcaaag aggcaacttg gtactacaac 1860

ggttataaac ctagtatgca agaatacctt aacaatgcat ggatatcagt cggaagtatg 1920

gctatactca accacctctt cttccggttc acaaacgaga gaatgcataa ataccgcgat 1980

atgaaccgtg tctcgtccaa cattgtgagg cttgctgatg atatgggaac atcattggct 2040

gaggtggaga gaggggacgt gccgaaagca attcaatgct acatgaatga gacgaatgct 2100

tctgaagaag aagcaagaga atatgtaaga agagtcatac aggaagaatg ggaaaagttg 2160

aacacagaat tgatgcggga tgatgatgat gatgatgatt ttacactatc caaatattac 2220

tgtgaggtgg ttgctaatct tacaagaatg gcacagttta tataccaaga tggatcggat 2280

ggcttcggca tgaaagattc caaggttaat agactgctaa aagagacgtt gatcgagcgc 2340

tacgaataat aatttaaaag caaatataaa tgaaaaattg aaccctagca ttatgtaaat 2400

gcagggttta atttttatat taagcagcat aatagaaagt tttttaaatg catgtatata 2460

tggggtattt aaagggaaat ctataatata atttaggact attggtacc 2509

<210> 15
<211> 7244
<212> DNA
<213> Artificial

<220>
<223> Plasmid vector pCM80

<400> 15
gaccctttcc gacgctcacc gggctggttg ccctcgccgc tgggctggcg gccgtctatg 60

gccctgcaaa cgcgccagaa acgccgtcga agccgtgtgc gagacaccgc ggccgccggc 120

gttgtggata cctcgcggaa aacttggccc tcactgacag atgaggggcg gacgttgaca 180

cttgaggggc cgactcaccc ggcgcggcgt tgacagatga ggggcaggct cgatttcggc 240

cggcgacgtg gagctggcca gcctcgcaaa tcggcgaaaa cgcctgattt tacgcgagtt 300

tcccacagat gatgtggaca agcctgggga taagtgccct gcggtattga cacttgaggg 360

gcgcgactac tgacagatga ggggcgcgat ccttgacact tgaggggcag agtgctgaca 420

gatgaggggc gcacctattg acatttgagg ggctgtccac aggcagaaaa tccagcattt 480

gcaagggttt ccgcccgttt ttcggccacc gctaacctgt cttttaacct gcttttaaac 540

caatatttat aaaccttgtt tttaaccagg gctgcgccct gtgcgcgtga ccgcgcacgc 600

cgaagggggg tgccccccct tctcgaaccc tcccggcccg ctaacgcggg cctcccatcc 660

ccccaggggc tgcgcccctc ggccgcgaac ggcctcaccc caaaaatggc agccaagctg 720

accacttctg cgctcggccc ttccggctgg ctggtttatt gctgataaat ctggagccgg 780

tgagcgtggg tctcgcggta tcattgcagc actggggcca gatggtaagc cctcccgtat 840

cgtagttatc tacacgacgg ggagtcaggc aactatggat gaacgaaata gacagatcgc 900

tgagataggt gcctcactga ttaagcattg gtaactgtca gaccaagttt actcatatat 960

actttagatt gatttaaaac ttcattttta atttaaaagg atctaggtga agatcctttt 1020

tgataatctc atgaccaaaa tcccttaacg tgagttttcg ttccactgag cgtcagaccc 1080

cgtagaaaag atcaaaggat cttcttgaga tccttttttt ctgcgcgtaa tctgctgctt 1140

gcaaacaaaa aaaccaccgc taccagcggt ggtttgtttg ccggatcaag agctaccaac 1200

tctttttccg aaggtaactg gcttcagcag agcgcagata ccaaatactg tccttctagt 1260

gtagccgtag ttaggccacc acttcaagaa ctctgtagca ccgcctacat acctcgctct 1320

gctaatcctg ttaccagtgg ctgctgccag tggcgataag tcgtgtctta ccgggttgga 1380

ctcaagacga tagttaccgg ataaggcgca gcggtcgggc tgaacggggg gttcgtgcac 1440

acagcccagc ttggagcgaa cgacctacac cgaactgaga tacctacagc gtgagctatg 1500

agaaagcgcc acgcttcccg aagggagaaa ggcggacagg tatccggtaa gcggcagggt 1560

cggaacagga gagcgcacga gggagcttcc agggggaaac gcctggtatc tttatagtcc 1620

tgtcgggttt cgccacctct gacttgagcg tcgatttttg tgatgctcgt caggggggcg 1680

gagcctatgg aaaaacgcca gcaacgcggc ctttttacgg ttcctggcct tttgctggcc 1740

ttttgctcac atgttctttc ctgcgttatc ccctgattct gtggataacc gtattaccgc 1800

ctttgagtga gctgataccg ctcgccgcag ccgaacgacc gagcgcagcg agtcagtgag 1860

cgaggaagcg gaagagcgcc caatacgcaa accgcctctc cccgcgcgtt ggccgattca 1920

ttaatgcagc tggcacgaca ggtttcccga ctggaaagcg ggcagtgagc gcaacgcaat 1980

taatgtgagt tagctcactc attaggcacc ccaggcttta cactttatgc ttccggctcg 2040

tatgttgtgt ggaattgtga gcggataaca atttcacaca ggaaacagct atgaccatga 2100

ttacgccaag ctagcttccc gcttggtcgg gccgcttcgc gagggcccgt tgacgacaac 2160

ggtgcgatgg gtcccggccc cggtcaagac gatgccaata cgttgcgaca ctacgccttg 2220

gcacttttag aattgcctta tcgtcctgat aagaaatgtc cgaccagcta aagacatcgc 2280

gtccaatcaa agcctagaaa atataggcga agggacgcta ataagtcttt cataagaccg 2340

cgcaaatcta aaaatatcct tagattcacg atgcggcact tcggatgact tccgagcgag 2400

cctggaacct cagaaaaacg tctgagagat accgcggatc tcacacagga aacagctatg 2460

accatgatta cgccaagctt gcatgcctgc aggtcgactc tagaggatcc ccgggtaccg 2520

agctcgaatt cactggccgt cgttttacaa cgtcgtgact gggaaaaccc tggcgttacc 2580

caacttaatc gccttgcagc acatccccct ttcgccagct ggcgtaatag cgaagaggcc 2640

cgcaccgatc gcccttccca acagttgcgc agcctgaatg gcgaatggcg cctgatgcgg 2700

tattttctcc ttacgcatct gtgcggtatt tcacaccgca tatggtgcac tctcagtaca 2760

atctgctctg atgccgcata gttaagccag ccccgacacc cgccaacacc cgctgacgcg 2820

ccctgacggg cttgtctgct cccggcatcc gcttacagac aagctgtgac cgtctccggg 2880

agctgcatgt gtcagaggtt ttcaccgtca tcaccgaaac gcgcgagacg aaagggcctc 2940

gtgatacgcc tatttttata ggttaatgtc atgataataa tggtttctta gcaccctttc 3000

tcggtccttc aacgttcctg acaacgagcc tccttttcgc caatccatcg acaatcaccg 3060

cgagtccctg ctcgaacgct gcgtccggac cggcttcgtc gaaggcgtct atcgcggccc 3120

gcaacagcgg cgagagcgga gcctgttcaa cggtgccgcc gcgctcgccg gcatcgctgt 3180

cgccggcctg ctcctcaagc acggccccaa cagtgaagta gctgattgtc atcagcgcat 3240

tgacggcgtc cccggccgaa aaacccgcct cgcagaggaa gcgaagctgc gcgtcggccg 3300

tttccatctg cggtgcgccc ggtcgcgtgc cggcatggat gcgcgcgcca tcgcggtagg 3360

cgagcagcgc ctgcctgaag ctgcgggcat tcccgatcag aaatgagcgc cagtcgtcgt 3420

cggctctcgg caccgaatgc gtatgattct ccgccagcat ggcttcggcc agtgcgtcga 3480

gcagcgcccg cttgttcctg aagtgccagt aaagcgccgg ctgctgaacc cccaaccgtt 3540

ccgccagttt gcgtgtcgtc agaccgtcta cgccgacctc gttcaacagg tccagggcgg 3600

cacggatcac tgtattcggc tgcaactttg tcatgattga cactttatca ctgataaaca 3660

taatatgtcc accaacttat cagtgataaa gaatccgcgc gttcaatcgg accagcggag 3720

gctggtccgg aggccagacg tgaaacccaa catacccctg atcgtaattc tgagcactgt 3780

cgcgctcgac gctgtcggca tcggcctgat tatgccggtg ctgccgggcc tcctgcgcga 3840

tctggttcac tcgaacgacg tcaccgccca ctatggcatt ctgctggcgc tgtatgcgtt 3900

ggtgcaattt gcctgcgcac ctgtgctggg cgcgctgtcg gatcgtttcg ggcggcggcc 3960

aatcttgctc gtctcgctgg ccggcgccac tgtcgactac gccatcatgg cgacagcgcc 4020

tttcctttgg gttctctata tcgggcggat cgtggccggc atcaccgggg cgactggggc 4080

ggtagccggc gcttatattg ccgatatcac tgatggcgat gagcgcgcgc ggcacttcgg 4140

cttcatgagc gcctgtttcg ggttcgggat ggtcgcggga cctgtgctcg gtgggctgat 4200

gggcggtttc tccccccacg ctccgttctt cgccgcggca gccttgaacg gcctcaattt 4260

cctgacgggc tgtttccttt tgccggagtc gcacaaaggc gaacgccggc cgttacgccg 4320

ggaggctctc aacccgctcg cttcgttccg gtgggcccgg ggcatgaccg tcgtcgccgc 4380

cctgatggcg gtcttcttca tcatgcaact tgtcggacag gtgccggccg cgctttgggt 4440

cattttcggc gaggatcgct ttcactggga cgcgaccacg atcggcattt cgcttgccgc 4500

atttggcatt ctgcattcac tcgcccaggc aatgatcacc ggccctgtag ccgcccggct 4560

cggcgaaagg cgggcactca tgctcggaat gattgccgac ggcacaggct acatcctgct 4620

tgccttcgcg acacggggat ggatggcgtt cccgatcatg gtcctgcttg cttcgggtgg 4680

catcggaatg ccggcgctgc aagcaatgtt gtccaggcag gtggatgagg aacgtcaggg 4740

gcagctgcaa ggctcactgg cggcgctcac cagcctgacc tcgatcgtcg gacccctcct 4800

cttcacggcg atctatgcgg cttctataac aacgtggaac gggtgggcat ggattgcagg 4860

cgctgccctc tacttgctct gcctgccggc gctgcgtcgc gggctttgga gcggcgcagg 4920

gcaacgagcc gatcgctgat cgtggaaacg ataggcctat gccatgcggg tcaaggcgac 4980

ttccggcaag ctatacgcgc cctagaattg tcaattttaa tcctctgttt atcggcagtt 5040

cgtagagcgc gccgtgcgtc ccgagcgata ctgagcgaag caagtgcgtc gagcagtgcc 5100

cgcttgttcc tgaaatgcca gtaaagcgct ggctgctgaa cccccagccg gaactgaccc 5160

cacaaggccc tagcgtttgc aatgcaccag gtcatcattg acccaggcgt gttccaccag 5220

gccgctgcct cgcaactctt cgcaggcttc gccgacctgc tcgcgccact tcttcacgcg 5280

ggtggaatcc gatccgcaca tgaggcggaa ggtttccagc ttgagcgggt acggctcccg 5340

gtgcgagctg aaatagtcga acatccgtcg ggccgtcggc gacagcttgc ggtacttctc 5400

ccatatgaat ttcgtgtagt ggtcgccagc aaacagcacg acgatttcct cgtcgatcag 5460

gacctggcaa cgggacgttt tcttgccacg gtccaggacg cggaagcggt gcagcagcga 5520

caccgattcc aggtgcccaa cgcggtcgga cgtgaagccc atcgccgtcg cctgtaggcg 5580

cgacaggcat tcctcggcct tcgtgtaata ccggccattg atcgaccagc ccaggtcctg 5640

gcaaagctcg tagaacgtga aggtgatcgg ctcgccgata ggggtgcgct tcgcgtactc 5700

caacacctgc tgccacacca gttcgtcatc gtcggcccgc agctcgacgc cggtgtaggt 5760

gatcttcacg tccttgttga cgtggaaaat gaccttgttt tgcagcgcct cgcgcgggat 5820

tttcttgttg cgcgtggtga acagggcaga gcgggccgtg tcgtttggca tcgctcgcat 5880

cgtgtccggc cacggcgcaa tatcgaacaa ggaaagctgc atttccttga tctgctgctt 5940

cgtgtgtttc agcaacgcgg cctgcttggc ctcgctgacc tgttttgcca ggtcctcgcc 6000

ggcggttttt cgcttcttgg tcgtcatagt tcctcgcgtg tcgatggtca tcgacttcgc 6060

caaacctgcc gcctcctgtt cgagacgacg cgaacgctcc acggcggccg atggcgcggg 6120

cagggcaggg ggagccagtt gcacgctgtc gcgctcgatc ttggccgtag cttgctggac 6180

catcgagccg acggactgga aggtttcgcg gggcgcacgc atgacggtgc ggcttgcgat 6240

ggtttcggca tcctcggcgg aaaaccccgc gtcgatcagt tcttgcctgt atgccttccg 6300

gtcaaacgtc cgattcattc accctccttg cgggattgcc ccgactcacg ccggggcaat 6360

gtgcccttat tcctgatttg acccgcctgg tgccttggtg tccagataat ccaccttatc 6420

ggcaatgaag tcggtcccgt agaccgtctg gccgtccttc tcgtacttgg tattccgaat 6480

cttgccctgc acgaatacca gctccgcgaa gtcgctcttc ttgatggagc gcatggggac 6540

gtgcttggca atcacgcgca ccccccggcc gttttagcgg ctaaaaaagt catggctctg 6600

ccctcgggcg gaccacgccc atcatgacct tgccaagctc gtcctgcttc tcttcgatct 6660

tcgccagcag ggcgaggatc gtggcatcac cgaaccgcgc cgtgcgcggg tcgtcggtga 6720

gccagagttt cagcaggccg cccaggcggc ccaggtcgcc attgatgcgg gccagctcgc 6780

ggacgtgctc atagtccacg acgcccgtga ttttgtagcc ctggccgacg gccagcaggt 6840

aggcctacag gctcatgccg gccgccgccg ccttttcctc aatcgctctt cgttcgtctg 6900

gaaggcagta caccttgata ggtgggctgc ccttcctggt tggcttggtt tcatcagcca 6960

tccgcttgcc ctcatctgtt acgccggcgg tagccggcca gcctcgcaga gcaggattcc 7020

cgttgagcac cgccaggtgc gaataaggga cagtgaagaa ggaacacccg ctcgcgggtg 7080

ggcctacttc acctatcctg cccggctgac gccgttggat acaccaagga aagtctacac 7140

gaaccctttg gcaaaatcct gtatatcgtg cgaaaaagga tggatatacc gaaaaaatcg 7200

ctataatgac cccgaagcag ggttatgcag cggaaaagat ccgt 7244

<210> 16
<211> 1963
<212> DNA
<213> Artificial

<220>
<223> Synthetic gene

<400> 16
ggatccttca gattaaattt ttgcttattt gatttacatt atataatatt gagtaaagta 60

ttgactagca aaattttttg atactttaat ttgtgaaatt tcttatcaaa agttatattt 120

ttgaatgatt tttattgaaa aatacaacta aaaaggatta tagtataagt gtgtgtaatt 180

ttgtgttaaa tttaaaggga ggaaatgaac atgacgggcc ggcgatccgg aggctacccg 240

cccgccctat gggatttcga cactattcaa tcgctcaaca ccgagtataa gggagagagg 300

cacatgagaa gggaagaaga cctaattggg caagttagag agatgctggt gcatgaagta 360

gaagatccca ctccacagct ggagttcatt gatgatttgc ataagcttgg catatcttgc 420

cattttgaga atgaaatcct ccaaatcttg aaatccatat atcttaatca aaactacaaa 480

agggatttgt actcaacatc tctagcattc agactcctca gacaatatgg cttcatcctt 540

ccacaagaag tatttgattg tttcaagaat gaggagggta cggatttcaa gccaagcttc 600

ggccgtgata tcaaaggctt gttacaattg tatgaagctt ctttcctatc aagaaaagga 660

gaagaaactt tacaactagc aagagagttt gcaacaaaga ttctgcaaaa agaagttgat 720

gagagagagt ttgcaaccaa gatggagttc ccttctcatt ggacggttca aatgccgaat 780

gcaagacctt tcatcgatgc ttaccgtagg aggccggata tgaatccagt tgtgctcgag 840

ctagccatac ttgatacaaa tatagttcaa gcacaatttc aagaagaact caaagagacc 900

tcaaggtggt gggagagtac aggcattgtc caagagcttc catttgtgag ggataggatt 960

gtggaaggct acttttggac gattggagtg actcagagac gcgagcatgg atacgaaaga 1020

atcatgaccg caaaggttat tgccttagta acatgtttag acgacatata cgatgtttat 1080

ggcacgatag aagagcttca acttttcaca agcacaatcc aaagatggga tttggaatca 1140

atgaagcaac tccctaccta catgcaagta agctttcttg cactacacaa ctttgtaacc 1200

gaggtggctt acgatactct caagaaaaag ggctacaact ccacaccata tttaagaaaa 1260

acgtgggtgg atcttgttga atcatatatc aaagaggcaa cttggtacta caacggttat 1320

aaacctagta tgcaagaata ccttaacaat gcatggatat cagtcggaag tatggctata 1380

ctcaaccacc tcttcttccg gttcacaaac gagagaatgc ataaataccg cgatatgaac 1440

cgtgtctcgt ccaacattgt gaggcttgct gatgatatgg gaacatcatt ggctgaggtg 1500

gagagagggg acgtgccgaa agcaattcaa tgctacatga atgagacgaa tgcttctgaa 1560

gaagaagcaa gagaatatgt aagaagagtc atacaggaag aatgggaaaa gttgaacaca 1620

gaattgatgc gggatgatga tgatgatgat gattttacac tatccaaata ttactgtgag 1680

gtggttgcta atcttacaag aatggcacag tttatatacc aagatggatc ggatggcttc 1740

ggcatgaaag attccaaggt taatagactg ctaaaagaga cgttgatcga gcgctacgaa 1800

taataattta aaagcaaata taaatgaaaa attgaaccct agcattatgt aaatgcaggg 1860

tttaattttt atattaagca gcataataga aagtttttta aatgcatgta tatatggggt 1920

atttaaaggg aaatctataa tataatttag gactattggt acc 1963